# FASCICULE DE BREVET EUROPEEN

(11) **EP 1 283 957 B1**
(45) Date de publication et mention de la délivrance du brevet: **26.10.2005**
(21) Numéro de dépôt: 01951621.0
(22) Date de dépôt: 25.05.2001
(51) Int. Cl.: F04B 43/04

(54) **DISPOSITIF FLUIDIQUE MICRO-USINE ET SON PROCEDE DE FABRICATION**
MICROBEARBEITETE FLUIDISCHE VORRICHTUNG UND HERSTELLUNGSVERFAHREN
MICROMACHINED FLUIDIC DEVICE AND METHOD FOR MAKING SAME

(30) Priorité: 25.05.2000 FR 0006669
(43) Date de publication de la demande: 19.02.2003
(73) Titulaire: DEBIOTECH S.A., 1004 Lausanne (CH)
(72) Inventeur: VAN LINTEL, Harald, T., CH-1006 Lausanne (CH); MAILLEFER, Didier, CH-1092 Belmont S/Lausanne (CH); GAMPER, Stephan, CH-1338 Pallaiques (CH)
(74) Mandataire: Cardy, Sophie Marie
(86) Numéro de dépôt international: PCT/EP2001/007032
(87) Numéro de publication internationale: WO 2001/090577

(56) Documents cités:
- EP-A- 0 483 469
- EP-A- 0 568 902
- WO-A-95/18307
- WO-A-97/29538
- DE-A- 4 143 343
- DE-A- 19 719 862
- GRAVESEN P ET AL: "MICROFLUIDICS - A REVIEW" JOURNAL OF MICROMECHANICS & MICROENGINEERING,NEW YORK, NY,US, vol. 3, 1993, pages 168-182, XP000601274 ISSN: 0960-1317

## Description

L'invention concerne un dispositif fluidique et son procédé de fabrication. L'invention concerne également des formes particulières de dispositifs fluidiques constituant un organe de contrôle d'entrée de liquide, qui forme par exemple une valve anti-retour, ou un organe de détection de pression de liquide.

La présente invention concerne aussi une micropompe constituant un dispositif fluidique et qui notamment, mais non exclusivement, forme une micropompe à usage médical qui délivre régulièrement une quantité contrôlée d'un médicament liquide.

La fabrication de ces dispositifs fluidiques et notamment de ces micropompes est basée sur les technologies de micro-usinage du silicium ou de tout autre matériau micro-usinable, notamment à l'aide de techniques photolithographiques et d'attaque chimique, d'ablation laser, de micro-réplication ou autres.

Pour l'application particulière précitée, et dans d'autres cas encore, il est nécessaire de réaliser un organe de contrôle d'entrée permettant l'auto-amorçage de la micropompe. La commande de la micropompe s'effectue en faisant varier le volume de la chambre de pompage (altemance de diminutions et d'augmentations), par exemple au moyen d'une commande réalisée par un actionneur piézoélectrique.

La demande de brevet européen publiée sous le n° 453 532 ou la demande de brevet allemand DE 197 19 862 A1 présentent une telle micropompe. Toutefois, une telle micropompe n'assure pas son auto-amorçage car elle présente d'importants volumes morts, ces volumes contribuant à dégrader le taux de compression atteint par la micropompe.

Afin d'améliorer cet aspect, a notamment été développée une nouvelle micropompe telle que décrite dans la demande intemationale publiée sous le n° WO 99/09321. Dans cette micropompe, afin de minimiser les volumes morts, notamment en aval du siège de la valve d'entrée, on réalise une valve d'entrée épaisse du fait que cette valve d'entrée constitue toute l'épaisseur de la plaque intermédiaire, le siège de la valve étant situé du côté opposé à la membrane mobile. Toutefois, une telle micropompe présente une structure complexe, de fabrication difficile, qui comporte des volumes morts toujours importants.

La présente invention a pour but de foumir un dispositif fluidique, par exemple un organe de contrôle d'entrée de liquide ou un organe de détection de pression de liquide ou une micropompe, pouvant être fabriqué de manière simplifiée et constituant un dispositif fluidique fiable dans son fonctionnement en minimisant les volumes morts.

A cet effet, selon l'invention, est proposé un dispositif fluidique comprenant les caractéristiques techniques selon la revendication 2. Selon un premier type de dispositif fluidique ledit organe mobile se rapproche, de manière réversible, de ladite plaque de support jusqu'à former un contact entre ledit organe mobile et ladite plaque de support.

Selon des caractéristiques préférentielles:
- ladite plaque (ou « wafer ») de support est en silicium, en quartz ou en saphir et présente une épaisseur comprise entre 50 µm et 1 mm, de préférence entre 300 µm et 500 µm;
- ladite couche de matériau isolant présente une épaisseur comprise entre 100 nm et 2 µm, de préférence entre 0,5 µm et 1 µm;
- ladite plaque de fermeture est en verre ou en silicium, de préférence monocristallin; et
- ladite couche de silicium est en silicium monocristallin ou polycristallin et présente une épaisseur comprise entre 1 µm et 100 µm, de préférence entre 10 µm et 50 µm.

Selon un autre but de la présente invention, on propose un procédé de fabrication selon la revendication 1, ce procédé de fabrication étant caractérisé en ce qu'il comporte les étapes suivantes :
- on fournit un empilement comprenant une plaque de support, une couche de matériau isolant, de préférence en oxyde de silicium, recouvrant au moins partiellement ladite plaque de support, et une couche de silicium monocristallin ou polycristallin recouvrant ladite couche de matériau isolant et présentant une face libre,
- on usine ladite cavité à partir de ladite plaque de fermeture et/ou de la face libre de ladite couche de silicium par photolithographie et attaque chimique,
- on usine au moins un conduit traversant de part en part ladite plaque de support par photolithographie et attaque chimique,
- on réalise une attaque chimique de ladite couche de matériau isolant au moins par ledit conduit de sorte qu'une zone de ladite couche de silicium est libérée de ladite couche de matériau isolant en formant ledit organe mobile,
- on fournit au moins une plaque de fermeture,
- on relie de façon étanche par un procédé physico-chimique, de préférence par soudage de plaques (ou « wafer bonding »), ladite plaque de fermeture à ladite surface de la couche de silicium qui n'a pas été usinée.

Ainsi, selon la présente invention, on utilise de préférence un empilement comportant une plaque de support en silicium recouverte d'une couche d'oxyde de silicium, elle-même recouverte d'une couche de silicium. Cet empilement est disponible dans le commerce et est habituellement dénommé SOI ("SILICON-ON-INSULATOR").

L'utilisation de ce type d'empilement permet d'obtenir un dispositif fluidique présentant des épaisseurs constantes et strictement contrôlées, tout en mettant en oeuvre un procédé de fabrication simple.

En effet, ce procéde de fabrication est notamment plus simple à mettre en oeuvre que celui présenté la demande internationale publiée sous le n°WO 98/14704.

La formation des différentes parties du dispositif fluidique est réalisée par attaque chimique sélective depuis les deux côtés de l'empilement, c'est-à-dire depuis les deux faces de la plaque constituant cet empilement: en effet, la couche de matériau isolant (en oxyde de silicium dans le cas d'un empilement SOI) forme un arrêt ou une barrière d'attaque lors du micro-usinage de la plaque de support ou de la couche de silicium.

En outre, la plaque de fermeture, qui permet de refermer notamment la cavité créée dans la couche de silicium usinée, est de préférence réalisée en verre ou en silicium monocristallin.

Dans le cas d'une plaque de fermeture en verre, sa fixation à la couche de silicium s'effectue de manière par ailleurs connue en soi selon la technique de soudage anodique.

Dans le cas d'une plaque de fermeture en silicium, sa fixation à la couche de silicium s'effectue selon la technique connue de soudage direct Si-Si.

Selon un premier aspect du dispositif fluidique selon l'invention, il est proposé un organe de contrôle d'entrée de liquide formant une valve anti-retour, comprenant un empilement recouvert d'une plaque de fermeture, ledit empilement comportant une plaque de support, de préférence en silicium, une couche de matériau isolant, de préférence en oxyde de silicium, recouvrant au moins partiellement ladite plaque de support, et une couche de silicium monocristallin ou polycristallin recouvrant ladite couche de matériau isolant et recouverte de ladite plaque de fermeture, ladite plaque de fermeture et/ou ladite couche de silicium étant usinée afin de définir, entre ladite plaque de fermeture et ladite couche de silicium, une cavité destinée à être remplie de liquide, ladite cavité présentant au moins un dégagement usiné dans toute l'épaisseur de la couche de silicium, ladite plaque de support comportant au moins un conduit d'entrée de liquide la traversant de part en part et situé au moins en regard de ladite cavité, ladite couche de matériau isolant ayant au moins une zone entièrement exempte de matière s'étendant au moins dans le prolongement dudit conduit et dudit dégagement, afin de définir, avec ladite cavité et dans ladite couche de silicium, un organe mobile formant un clapet pour ladite valve, une partie de ladite couche de silicium entourant ledit organe mobile permettant, par son élasticité et par la différence de pression de liquide entre ledit conduit d'entrée de liquide et ladite cavité, le mouvement réversible dudit organe mobile en direction de ladite plaque de support.

Selon le premier type de dispositif fluidique, ledit conduit d'entrée de liquide de l'organe de contrôle d'entrée de liquide défini dans le paragraphe qui précède, est situé à proximité mais pas au regard dudit dégagement, ledit organe mobile réalise un mouvement entre une position fermée, dans laquelle l'organe mobile est en contact étanche contre ladite plaque de support qui forme, au moins autour dudit conduit, un siège pour ladite valve, l'écoulement de liquide étant empêché entre ledit conduit d'entrée de liquide et la cavité, et une position ouverte de la valve dans laquelle, l'organe mobile n'étant plus en contact étanche contre la plaque de support autour dudit conduit, l'organe mobile permet l'écoulement du liquide depuis ledit conduit d'entrée de liquide vers ledit dégagement.

Selon un deuxième aspect du dispositif fluidique selon la présente invention, il est proposé un organe de détection de pression de liquide comprenant un empilement recouvert d'une plaque de fermeture, ledit empilement comportant une plaque de support, de préférence en silicium, une couche de matériau isolant, de préférence en oxyde de silicium, recouvrant au moins partiellement ladite plaque de support, et une couche de silicium monocristallin ou polycristallin recouvrant ladite couche de matériau isolant et recouverte de ladite plaque de fermeture, ladite plaque de fermeture et/ou ladite couche de silicium étant usinée afin de définir, entre ladite plaque de fermeture et ladite couche de silicium, une cavité destinée à être remplie de liquide, ladite plaque de support comportant au moins un conduit la traversant de part en part et situé en regard de ladite cavité, ladite couche de matériau isolant ayant au moins une zone entièrement exempte de matière placée au moins dans le prolongement dudit conduit, afin de définir, avec ladite cavité et dans ladite couche de silicium, un organe mobile, ladite plaque de support en silicium présentant une partie en regard de l'organe mobile formant un îlot séparé du reste de la plaque de support par ledit conduit, ledit organe mobile étant susceptible, grâce à son élasticité et sous la pression de liquide dans ladite cavité, de se rapprocher de manière réversible de la plaque de support.

Selon le premier type de dispositif fluidique, ledit organe mobile, de l'organe de détection de pression de liquide défini dans le paragraphe qui précède, est susceptible de passer d'une position ouverte à une position fermée dans laquelle l'organe mobile est en contact physique avec ladite partie située en regard de l'organe mobile qui forme un îlot séparé du reste de la plaque de support par ledit conduit et qui constitue une partie d'appui de la plaque de silicium, ce contact physique pouvant être détecté électriquement.

Selon un troisième aspect du dispositif fluidique selon la présente invention, il est proposé également une micropompe comprenant un empilement recouvert d'une plaque de fermeture, ledit empilement comportant une plaque de support, de préférence en silicium, une couche de matériau isolant, de préférence en oxyde de silicium, recouvrant au moins partiellement ladite plaque de support, et une couche de silicium monocristallin ou polycristallin recouvrant ladite couche de matériau isolant et recouverte de ladite plaque de fermeture, ladite plaque de fermeture et/ou ladite couche de silicium étant usinée afin de définir, entre ladite plaque de fermeture et ladite couche de silicium, une cavité destinée à être remplie de liquide et comportant une chambre de pompage, ladite plaque de support comportant au moins un premier conduit la traversant de part en part et situé en regard de ladite cavité, ladite couche de matériau isolant ayant au moins une première zone entièrement exempte de matière placée au moins dans le prolongement dudit premier conduit, afin de définir, avec ladite cavité et dans ladite couche de silicium, un premier organe mobile apte, sous la pression de liquide dans ladite chambre de pompage, à se rapprocher de façon réversible de ladite plaque de support, ledit premier organe mobile appartenant au clapet d'un organe de contrôle d'entrée de liquide, ladite micropompe comprenant en outre une partie de pompage comprenant des moyens de commande munis d'une membrane de pompage, pour provoquer une variation périodique du volume de la chambre de pompage, et des moyens de contrôle de sortie du liquide.

Selon le premier type de dispositif fluidique, le premier organe mobile de l'organe de contrôle d'entrée de liquide défini dans le paragraphe qui précède, est apte, sous la pression de liquide dans ladite chambre de pompage, à venir en contact étanche contre ladite plaque de support, en constituant le clapet dudit organe de contrôle d'entrée de liquide.

De manière préférentielle, cette micropompe comprend, en outre, une deuxième zone entièrement exempte de matière dans ladite couche de matériau isolant qui définit, avec ladite cavité et dans ladite couche de silicium, un deuxième organe mobile apte, sous la pression de liquide dans ladite chambre de pompage, à se rapprocher de ladite plaque de support, ledit deuxième organe mobile constituant le clapet d'un organe de contrôle de sortie de liquide,

Ainsi la présente invention concerne différents types de dispositifs fluidiques qui sont, selon la caractéristique essentielle de la présente invention, réalisés à partir d'un empilement de type SOI, c'est-à-dire comportant une plaque de support, de préférence en silicium, recouverte d'une couche de matériau isolant, de préférence d'oxyde de silicium, elle-même recouverte d'une couche de silicium.

Ainsi, contrairement aux micropompes et aux dispositifs fluidiques de l'art antérieur pour lesquels il était nécessaire de réaliser l'usinage dans toute l'épaisseur d'une plaque en silicium destinée à former la plaque intermédiaire entre deux plaques de verre, la présente invention propose l'utilisation d'un empilement dont l'épaisseur initiale des trois constituants (plaque de support, couche de matériau isolant et couche de silicium) assure, d'une part, une épaisseur maîtrisée des différentes parties du dispositif fluidique, et, d'autre part, des volumes morts réduits de manière très importante par rapport à l'art antérieur.

Un autre avantage majeur de la technologie selon la présente invention est une simplification du procédé de fabrication par rapport aux techniques de l'art antérieur.

L'invention sera mieux comprise, et des caractéristiques secondaires et leurs avantages apparaîtront au cours de la description de différents modes de réalisation de l'invention donnée à titre d'exemple.

Il est entendu que la description et les dessins ne sont donnés qu'à titre indicatif et non limitatif.

Il sera fait référence aux dessins annexés, dans lesquels :
- la figure 1 représente une coupe transversale d'un organe de contrôle d'entrée de liquide, constituant le premier aspect d'un dispositif fluidique selon la présente invention, selon un premier mode de réalisation,
- les figures 1A et 1B représentent en vue de dessus deux variantes de l'organe de contrôle d'entrée de liquide de la figure 1, la plaque de fermeture recouvrant l'empilement ayant été retirée,
- les figures 2, 2A et 2B sont des vues similaires à celles des figures 1, 1A et 1B pour un deuxième mode de réalisation du premier aspect d'un dispositif fluidique selon la présente invention,
- les figures 3 et 3A sont des vues similaires à celles des figures 1 et 1A pour un troisième mode de réalisation du premier aspect d'un dispositif fluidique selon la présente invention,
- les figures 4 et 4A sont des vues similaires à celles des figures 1 et 1A pour un quatrième mode de réalisation du premier aspect d'un dispositif fluidique selon la présente invention,
- la figure 5 représente, en coupe transversale, un détecteur de pression de liquide, constituant le deuxième aspect d'un dispositif fluidique selon la présente invention,
- les figures 5A et 5B représentent en vue de dessus deux variantes de réalisation du détecteur de pression de liquide de la figure 5, la plaque de fermeture recouvrant l'empilement et la couche de silicium ayant été retirées,
- la figure 6 est une vue similaires à celle de la figure 5 pour un deuxième mode de réalisation du deuxième aspect d'un dispositif fluidique selon la présente invention,
- la figure 7 représente une vue en coupe longitudinale et en perspective partielle d'une micropompe selon un troisième aspect d'un dispositif fluidique selon la présente invention,
- la figure 8 est une vue en section longitudinale schématique de la micropompe de la figure 7,
- la figure 9 représente en vue de dessus la micropompe des figures 7 et 8, la plaque de fermeture recouvrant l'empilement ayant été retirée,
- les figures 10 à 15 représentent les différentes étapes de fabrication de la micropompe des figures 7 et 8,
- la figure 16A représente en vue de dessus une variante de réalisation de la partie de pompage des figures 7 à 9, qui forme plusieurs détecteurs de pression de liquide, la plaque de fermeture recouvrant l'empilement et la couche de silicium ayant été retirées,
- la figure 16B est une vue similaire à la figure 16A et représente une autre variante de réalisation de la partie de pompage des figures 7 à 9, qui forme un détecteur de pression de liquide de forme annulaire,
- la figure 17 est une vue agrandie de l'organe de contrôle de sortie de liquide de la micropompe des figures 7 et 8, et
- la figure 18 représente une variante de réalisation de l'organe de contrôle de sortie de liquide de la figure 17.

Sur ces figures, un élément identique représenté sur plusieurs figures porte toujours la même référence numérique.

En outre, pour des raisons de clarté, il doit être relevé que les épaisseurs des différents éléments représentés ont été très largement exagérées sur les dessins de sorte que ceux-ci ne sont pas strictement à l'échelle.

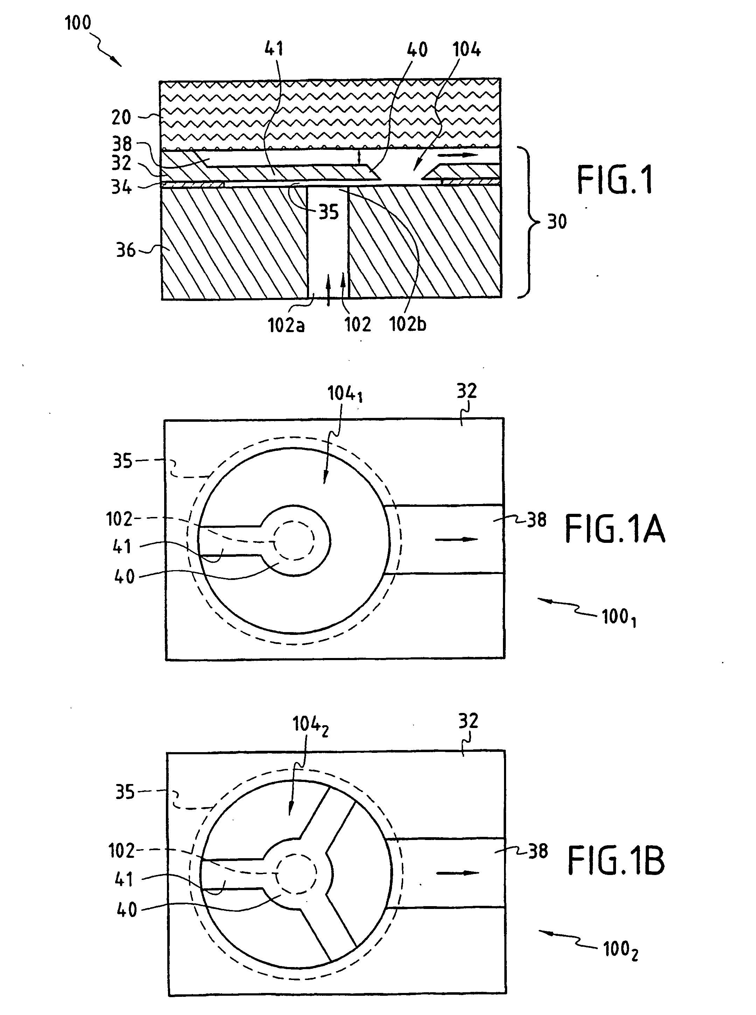

Selon un premier aspect du dispositif fluidique selon la présente l'invention, on considère un dispositif fluidique formant un organe de contrôle d'entrée de liquide dont un mode de réalisation est illustré sur la figure 1. Cet organe de contrôle d'entrée de liquide 100, destiné à former une valve unidirectionnelle ou valve anti-retour, comporte une plaque de fermeture de verre 20 disposée au-dessus d'un empilement 30 qui a été préalablement usiné pour la formation des différentes parties fonctionnelles de cet organe de contrôle 100.

Cet empilement 30 se compose d'une couche de silicium 32 surmontant une couche d'oxyde de silicium 34, elle-même disposée au-dessus d'une plaque de support en silicium 36.

Ce type d'empilement dénommé communément SOI («SILICON-ON-INSULATOR») est disponible dans le commerce sous la forme de plaques ou "wafer" notamment utilisés dans l'industrie électronique à semiconducteur. Le rôle de ces trois éléments de l'empilement 30 détermine des épaisseurs sensiblement différentes :
- la plaque de support en silicium 36 servant de base rigide, celle-ci présente de préférence une épaisseur située entre 50 et 1000 µm, avantageusement entre 300 et 500 µm,
- la couche d'oxyde de silicium 34 est destinée à relier la plaque de support en silicium 36 et la couche en silicium 32 en maintenant un écart constant entre elles, tout en pouvant être facilement être retirée dans certaines zones de sorte que son épaisseur doit rester très faible, de préférence entre 0,1 à 2 µm et avantageusement entre 0,5 et 1 µm, et
- la couche de silicium 32 est destinée à être usinée dans toute son épaisseur pour former des passages de liquide ou bien dans une partie seulement de son épaisseur (environ la moitié) afin de délimiter, avec la plaque de fermeture de verre 20, une cavité, et dans certains cas un organe mobile ;cette couche de silicium 32 , qui peut être réalisée dans du silicium monocristallin ou polycristallin, présente une épaisseur initiale de préférence comprise entre 1 et 100 µm, et avantageusement entre 10 et 50 µm.

L'empilement 30 est usiné par les techniques classiques de photolithographie et attaque chimique afin d'obtenir les différents éléments fonctionnels de l'organe de contrôle d'entrée 100, notamment une cavité 38 et un organe mobile 40, avant d'effectuer la liaison entre la plaque de fermeture de verre 20 et cet empilement 30. Cette liaison entre la plaque de fermeture 20 et la face libre de la couche de silicium 32 est, de manière connue, effectuée par soudage de plaque (soudage anodique lorsque la plaque de fermeture est en verre) à l'issue duquel on obtient une fixation sous la forme d'une liaison étanche.

Un conduit d'entrée de liquide 102 traverse la plaque de support en silicium dans toute son épaisseur depuis une première extrémité 102a jusqu'à une deuxième extrémité 102b. Cette deuxième extrémité 102b est adjacente à une zone circulaire 35 de la couche d'oxyde de silicium 34 entièrement exempte de matière qui s'étend bien au-delà du conduit d'entrée de liquide 102.

La couche de silicium 32 a été usinée dans une partie de son épaisseur du côté opposé à la plaque de support en silicium 36, afin de former la cavité 38. En outre, un dégagement 104, correspondant à un retrait de matière dans toute l'épaisseur de la couche de silicium 32, est situé dans le prolongement de la cavité 38 et de la zone 35 exempte d'oxyde de silicium, à proximité mais non directement en regard de la deuxième extrémité 102b du conduit d'entrée de liquide 102 qui est tournée vers la couche d'oxyde de silicium 34.

La cavité 38 s'étend au moins en regard de cette zone 35 et du conduit d'entrée de liquide 102.

De cette manière, comme il apparaît plus clairement sur les figures 1A et 1B, est formé un organe 40 dans la couche de silicium 32, cet organe 40 n'étant pas relié à la plaque de fermeture de verre 20, ni à la couche d'oxyde de silicium 34 et cet organe 40 étant séparé du reste de la couche de silicium 32 par le dégagement 104.

Selon une première variante de réalisation, l'organe de contrôle d'entrée de liquide 100₁ illustré sur la figure 1A comporte un dégagement 104₁ qui présente la forme d'un anneau ouvert de sorte que l'organe mobile est relié au reste de la couche de silicium 32 par un bras 41 situé à gauche de la figure 1A.

Selon une deuxième variante de réalisation, l'organe de contrôle d'entrée de liquide 100₂ illustré sur la figure 1B comporte un dégagement 104₂ qui présente la forme de trois secteur angulaires ayant un angle au centre de l'ordre de 120° de sorte que l'organe mobile est relié au reste de la couche de silicium 32 par trois bras 41 situés chacun entre deux des secteurs angulaires précités.

On comprend que l'organe 40 est mobile selon une direction perpendiculaire au plan principal de l'empilement 30, c'est-à-dire de bas en haut sur la figure 1 ou de manière orthogonale au plan de la feuille pour les figures 1A et 1B. Toutefois, dans le cas de la deuxième variante de réalisation, du fait des trois points d'attache de l'organe mobile 40 à la couche de silicium 32, on comprend que l'organe mobile 40 possède une rigidité plus grande que dans le cas de la première variante de réalisation.

La très faible épaisseur de cet organe 40 (moins de 50 µm et de préférence de l'ordre de 10 µm) le rend élastiquement mobile selon une direction transversale au plan principal de l'empilement 30 ou de la plaque de fermeture de verre 20, à savoir de haut en bas comme l'indique la flèche à double pointe de la figure 1.

Sur cette figure 1, l'organe 100 formant une valve d'entrée est illustré en position de repos, c'est-à-dire partiellement ouverte. Lorsque le liquide arrive par le conduit d'entrée 102, l'organe mobile 40 se soulève sous la pression de liquide qui est alors supérieure dans le conduit d'entrée 102 que dans la cavité 38, de sorte que la valve se place dans sa positon ouverte et permet au liquide de pénétrer dans la zone 35, de passer dans le dégagement 104 afin de parvenir dans la cavité 38.

Cet organe 100 est susceptible de s'insérer dans un ensemble fluidique plus complexe dans lequel l'organe 100 constitue un élément amont d'entrée du liquide. Ainsi, on comprend que le liquide présent dans la cavité 38 est susceptible de présenter une pression supérieure à la pression de liquide dans le conduit d'entrée 102, ce qui permet alors la fermeture de l'organe 100 par l'abaissement de l'organe mobile qui vient en contact étanche contre la face de la plaque de support en silicium 36 tournée en direction de la couche de silicium tout autour de la deuxième extrémité 102b du conduit 102.

L'élasticité relative de la couche de silicium 32 amincie à l'emplacement de la cavité 38 (le bras unique 41 de la figure 1 A ou les trois bras 41 de la figure 1B) permet, lorsque la pression de liquide dans la cavité 38 n'est plus supérieure à la pression de liquide dans le conduit 102, un retour de l'organe 40 dans sa position initiale telle que représentée sur la figure 1, c'est-à-dire une fermeture partielle de l'organe 100. Lorsque la pression de liquide dans la cavité 38 devient supérieure à la pression de liquide dans le conduit 102, l'organe mobile 40 s'abaisse complètement jusqu'à venir en contact étanche contre la plaque de support 36: l'organe de contrôle d'entrée de liquide 100 est alors fermé.

On comprend que cet organe 100 forme une valve d'entrée dans laquelle le corps de la valve est constitué de la face de l'organe mobile 40 tournée en direction de la plaque de support en silicium 36 et dans laquelle le siège de la valve est constitué par la région de la face de la plaque de support en silicium 36 tournée en direction de la couche de silicium 32 qui entoure la deuxième extrémité 102b du conduit 102.

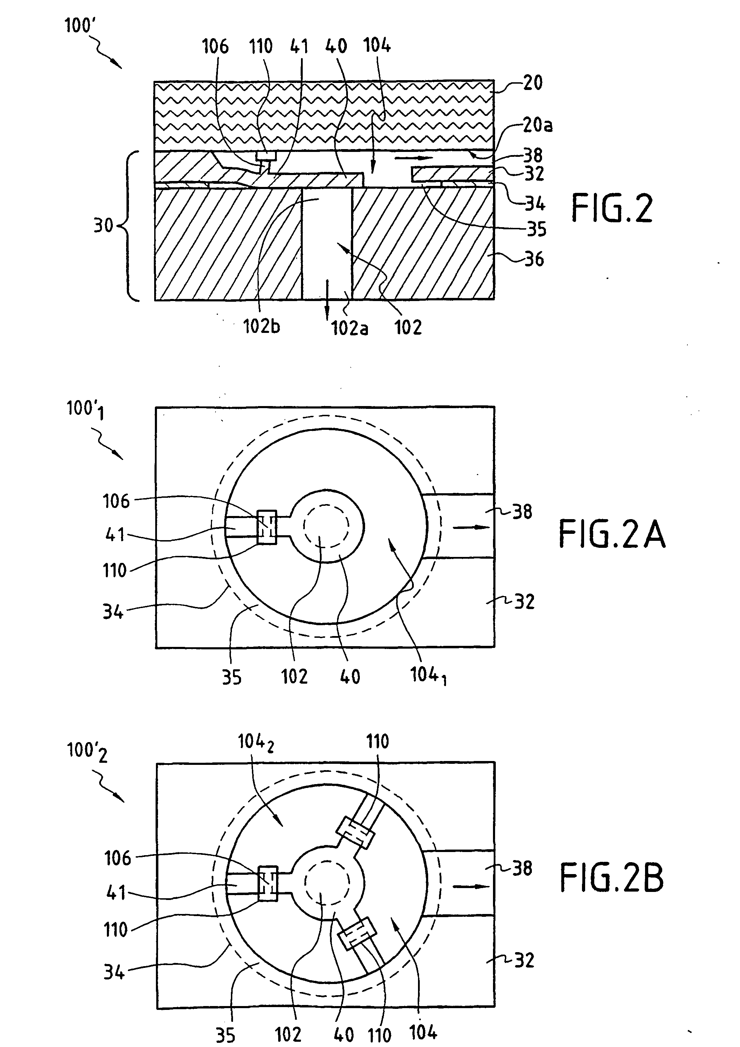

Selon un deuxième mode de réalisation illustré sur les figures 2,2A et 2B, il est également possible de créer une précontrainte qui place l'organe de contrôle d'entrée de liquide 100' en position fermée dans la position de repos de l'organe mobile 40.

A cet effet, une portion isolée 106 de l'organe mobile 40, située au milieu du bras 41, présente une épaisseur égale à l'épaisseur initiale de la couche de silicium 32. Cette portion 106 se trouve dans le prolongement d'un élément 110 situé sur la face 20a de la plaque de fermeture 20 tournée en direction de l'empilement ou sur la face libre de ladite portion 106.

De préférence, cet élément 110 est issu d'une couche en titane déposée sur la face 20a précitée de la plaque de fermeture 20. Cet élément 110 appuie vers le bas sur la portion isolée 106 et place de force l'organe mobile 40 dans sa position de fermeture correspondant alors à sa position de repos. L'élasticité de l'organe mobile 40 est toutefois suffisante pour permettre son ouverture.

Ainsi, la portion 106 et ledit élément 110 forment des moyens d'appui plaçant ledit organe mobile 40, dans sa position de repos, dans ladite position fermée.

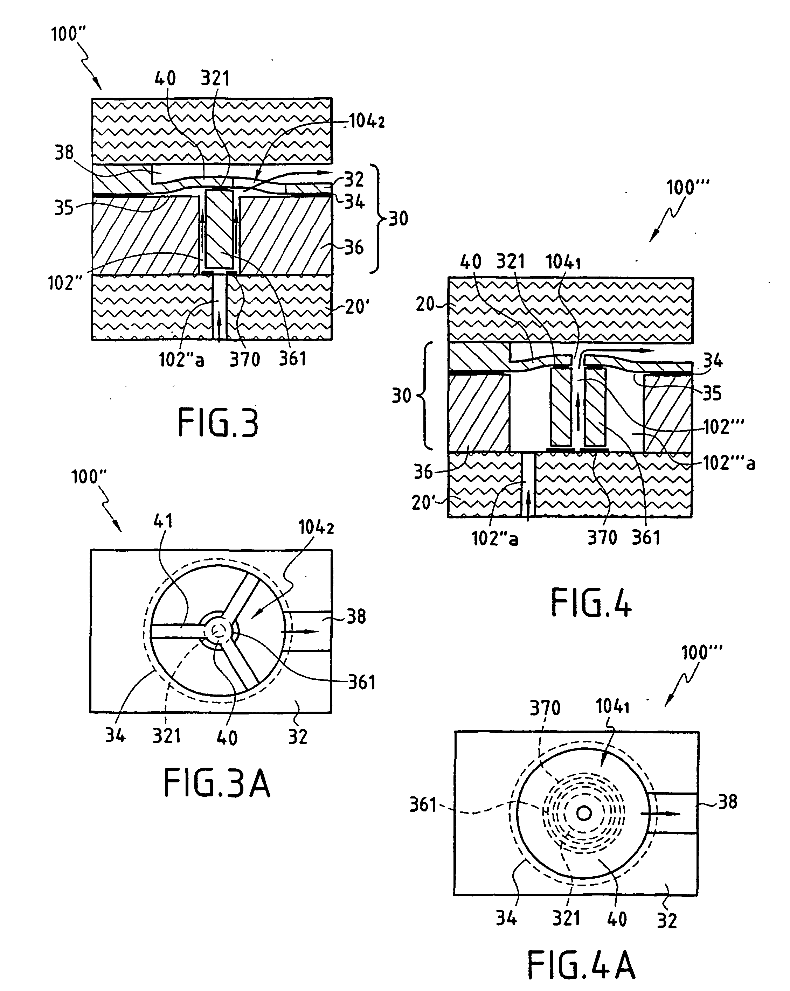

Les figures 3 et 3A concement un troisième mode de réalisation de l'organe de contrôle d'entrée de liquide constituant le premier aspect de l'invention. Dans ce cas, l'organe de contrôle d'entrée de liquide 100" comporte en outre, par rapport aux premier et deuxième modes de réalisation de l'organe de contrôle d'entrée de liquide, une deuxième plaque de verre 20'.

Ainsi, dans cet organe de contrôle d'entrée de liquide 100, la plaque de fermeture supérieure 20 est une première plaque de fermeture 20 en verre et la deuxième plaque en verre 20' forme une deuxième plaque de fermeture qui est fixée sur la face de la plaque de support 36 opposée à ladite première plaque de fermeture 20 en verre.

Cette deuxième plaque de fermeture 20' en verre est munie d'un conduit 102"a la traversant de part en part.

Pour déporter le corps et le siège de la valve entre la deuxième plaque de fermeture 20' et la plaque de support en silicium 36, une partie mobile 361 est réalisée dans toute l'épaisseur de la plaque de support 36, en regard et dans le prolongement de ladite cavité 38, dudit organe mobile 40 et dudit conduit 102"a. Cette partie mobile 361 est située à proximité mais pas au regard dudit dégagement 104₂.

Un volume annulaire 102" exempt de matière est usiné dans toute l'épaisseur de la plaque de support 36, en regard de ladite zone 35 entièrement exempte de matière de la couche de matériau isolant 34, en séparant ladite partie mobile 361 du reste de la plaque de support 36 et en formant ainsi ledit conduit d'entrée de liquide 102" de la plaque de support 36 qui communique avec ledit dégagement 104₂.

La couche de matériau isolant 34 présente une zone de liaison 321 entourée par la zone 35 qui est alors annulaire, la zone de liaison 321 reliant de manière solidaire ladite partie mobile 361 audit organe mobile 40, rendant ainsi la partie mobile 361 tributaire du mouvement ascendant ou descendant de l'organe mobile 40.

Un élément de valve 370 annulaire réalisé dans un matériau anti-adhésion (de préférence en titane) est situé sur la face de la deuxième plaque de fermeture 20' en verre placée en regard de ladite partie mobile 361.

Grâce à cet élément de valve 370, lorsque ledit organe mobile 40 est le plus près possible de la plaque de support 36 (situation non représentée), la face de la partie mobile 361 tournée vers la deuxième plaque de fermeture 20' et la face de l'élément de valve 370 tournée vers la plaque de support 36 sont en contact étanche mettant ainsi l'organe de contrôle d'entrée de liquide 100" en position de fermeture empêchant le passage de liquide depuis le conduit 102"a de la deuxième plaque de fermeture 20' vers ledit conduit d'entrée de liquide 102" de la plaque de support 36.

Au contraire, lorsqu'il n'y a pas de contact entre le corps de la valve (la partie mobile 361 dans le cas de figure illustré aux figures 3 et 3A) et le siège de la valve (l'élément de valve 370 dans le cas de figure illustré aux figures 3 et 3A), l'organe de contrôle d'entrée de liquide 100" est en position d'ouverture autorisant le passage de liquide depuis le conduit 102"a de la deuxième plaque de fermeture 20' vers ledit conduit d'entrée de liquide 102" de la plaque de support 36, puis vers le dégagement 104₂ en direction de la cavité 38. c'est la situation représentée sur la figure 3.

Si l'on se reporte à la figure 3A représentant l'organe de contrôle d'entrée de liquide 100" de la figure 3 en vue de dessus après que la plaque de fermeture 20 ait été enlevée, on s'aperçoit qu'il existe une similitude importante avec la figure 1B puisque l'organe mobile 40 est également relié par trois bras 41 au reste de la couche de silicium 32. Le dégagement 104₂ est ainsi également formé de trois secteurs angulaires ayant un angle au centre d'environ 120° et qui sont ici annulaires.

Les figures 4 et 4A concement un quatrième mode de réalisation de l'organe de contrôle d'entrée de liquide constituant le deuxième aspect de l'invention. Dans ce cas, comme pour le troisième mode de réalisation illustré aux figures 3 et 3A, l'organe de contrôle d'entrée de liquide 100"' comporte en outre, par rapport aux premier et deuxième modes de réalisation de l'organe de contrôle d'entrée de liquide, une deuxième plaque de verre 20'.

Cette deuxième plaque de verre 20' forme une deuxième plaque de fermeture en verre qui est fixée sur la face de la plaque de support 36 opposée à ladite première plaque de fermeture 20 en verre et munie d'un conduit 102"a la traversant de part en part.

Comme pour le troisième mode de réalisation de l'organe de contrôle d'entrée de liquide illustré aux figures 3 et 3A, également pour déporter le corps et le siège de la valve entre la deuxième plaque de fermeture 20' et la plaque de support en silicium, une partie mobile 361 est réalisée dans toute l'épaisseur de la plaque de support 36 de l'organe de contrôle d'entrée de liquide 100"', en regard et dans le prolongement de ladite cavité 38 et dudit organe mobile 40.

Cette partie mobile 361 est annulaire (voir les figures 4 et 4A) et est tout d'abord délimitée par un premier volume annulaire 102"'a exempt de matière qui a été usiné dans toute l'épaisseur de la plaque de support 36, en regard de ladite zone 35 entièrement exempte de matière de la couche de matériau isolant 34 et de ladite cavité 38. Ainsi, ce premier volume annulaire 102"'a sépare ladite partie mobile 361 du reste de la plaque de support 36.

Cette partie mobile 361 annulaire est ensuite également délimitée par un deuxième volume cylindrique exempt de matière 102"' qui a été usiné dans toute l'épaisseur de la plaque de support 36 à l'emplacement (au milieu) de la partie mobile 361. Ce deuxième volume exempt de matière 102"' forme ledit conduit d'entrée de liquide 102"' qui communique avec le dégagement 104₁ qui est alors situé en regard et dans le prolongement de ce conduit d'entrée de liquide 102"'.

Egalement comme pour le troisième mode de réalisation de l'organe de contrôle d'entrée de liquide 100" illustré aux figures 3 et 3A, la couche de matériau isolant 34 de l'organe de contrôle d'entrée de liquide 100"' selon le quatrième mode de réalisation présente une zone de liaison 321 entourée par la zone 35 et reliant de manière solidaire ladite partie mobile 361 audit organe mobile 40 autour du conduit d'entrée de liquide 102"' et du dégagement 104₁. Dans ce cas la zone de liaison 321 et la zone 35 sont annulaires et concentriques.

Cet organe de contrôle d'entrée de liquide 100"' comporte en outre un élément de valve 370 annulaire réalisé dans un matériau anti-adhésion (de préférence en titane) et situé sur la face de la deuxième plaque de fermeture 20' en verre placée en regard de ladite partie mobile 361.

Cet élément de valve 370 annulaire entoure ledit conduit d'entrée de liquide 102"' de la plaque de support 36 mais pas le conduit 102"a de la plaque de fermeture 20' qui débouche dans le premier volume annulaire 102"'a exempt de matière de la plaque de support 36.

Grâce à cet élément de valve 370, lorsque ledit organe mobile 40 est le plus près possible de la plaque de support 36 (situation non représentée), la face de la partie mobile 361 tournée vers la deuxième plaque de fermeture 20' et la face de l'élément de valve 370 tournée vers la plaque de support 36 sont en contact étanche mettant ainsi l'organe de contrôle d'entrée de liquide en position de fermeture. Dans cette position de fermeture de l'organe de contrôle d'entrée de liquide 100"' le liquide arrivant dans le premier volume annulaire 102"' depuis le conduit 102"a de la deuxième plaque de fermeture 20' ne peut pas pénétrer dans ledit conduit d'entrée de liquide 102"' de la plaque de support 36 : le liquide reste bloqué dans le premier volume annulaire 102"'a de la plaque de support 36.

Au contraire, lorsqu'il n'y a pas de contact entre le corps de la valve (la partie mobile 361 dans le cas de figure illustré aux figures 4 et 4A) et le siège de la valve (l'élément de valve 370 dans le cas de figure illustré aux figures 4 et 4A), l'organe de contrôle d'entrée de liquide 100"' est en position d'ouverture (voir les figures 4 et 4A) autorisant le passage de liquide depuis le conduit 102"a de la deuxième plaque de fermeture 20' vers le premier volume annulaire 102"'a de la plaque de support 36, puis entre la partie mobile 361 et l'élément de valve 370 vers le conduit d'entrée de liquide 102"' de la plaque de support 36, puis vers le dégagement 104₁ en direction de la cavité 38.

L'élément de valve 370 des troisième et quatrième modes de réalisation de l'organe de contrôle d'entrée de liquide (100" et 100"') pourrait aussi être situé sur la face de ladite partie mobile 361 placée en regard de la deuxième plaque de fermeture 20' en verre et/ou être réalisé également en un autre matériau anti-adhésion comme l'or, l'oxyde de silicium ou le nitrure de silicium.

Ainsi, les troisième et quatrième modes de réalisation de l'organe de contrôle d'entrée de liquide, respectivement illustrés sur les figures 3, 3A et 4, 4A, appartiennent à un deuxième type de dispositif fluidique dans lequel une deuxième plaque de verre 20' est nécessaire pour déporter le siège de la valve entre cette deuxième plaque de verre 20' et une partie mobile 361 de la plaque de support 36 de l'empilement 30.

Le fonctionnement des organes de contrôle d'entrée de liquide 100" ou 100"' est identique à celui des organes de contrôle d'entrée de liquide 100 et 100' selon les premier et deuxième modes de réalisation (respectivement figures 1, 1A, 1B et figures 2, 2A, et 2B).

Pour ce qui est du procédé de fabrication des organes de contrôle d'entrée de liquide 100" ou 100"', il suffit, par rapport au procédé de fabrication des organes de contrôle d'entrée de liquide 100 et 100' selon les premier et deuxième modes de réalisation (respectivement figures 1, 1A, 1B et figures 2, 2A, et 2B, de prévoir une deuxième plaque de fermeture 20' sur laquelle on aura déposé un élément de valve 370 annulaire réalisé dans un matériau anti-adhésion et de la relier à la plaque de support en silicium 36. Ces deux étapes seront réalisées à la fin du procédé de fabrication, c'est à dire après traitement (notamment par usinage et/ou structuration) de l'empilement 30.

La présence de l'élément de valve 370 dans les organes de contrôle d'entrée de liquide 100" ou 100"' permet de mettre en pré-tension l'organe mobile 40 car la présence de l'épaisseur de l'élément de valve 370 décale vers le haut d'autant (voir les figures 3 et 4) l'organe mobile 40 dans la cavité 38.

Grâce aux techniques de micro-usinage pouvant être employées pour traiter l'empilement 30, on peut contrôler très précisément le volume des conduits d'entrée de liquide 102" ou 102"' de la plaque de support 36 afin de minimiser le volume mort engendré par ces conduits.

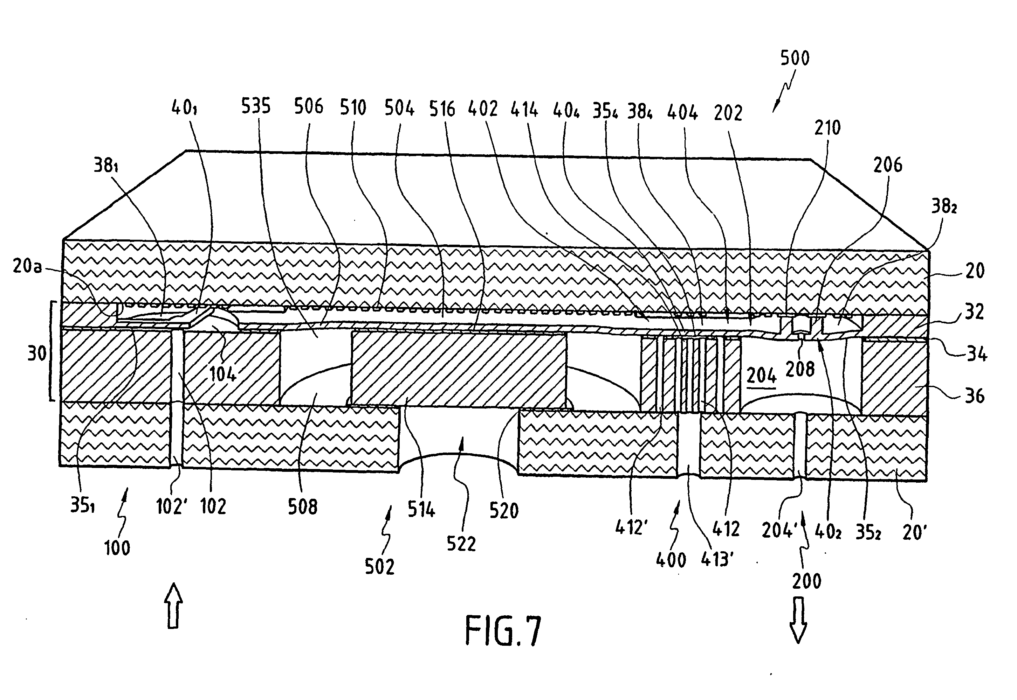

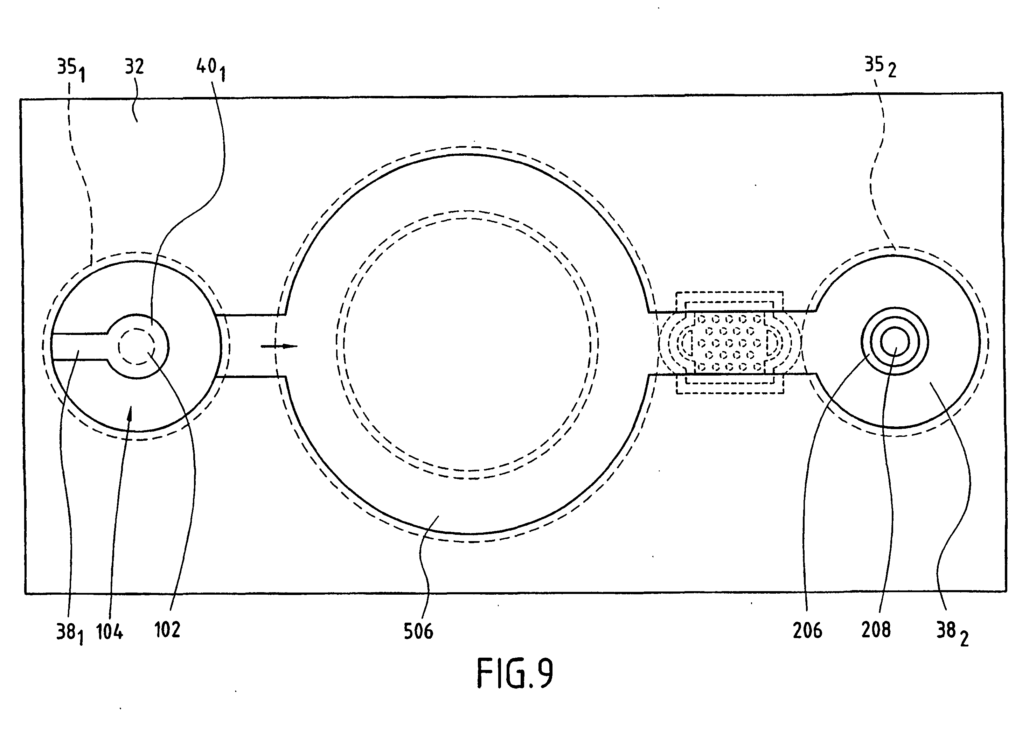

Les organes de contrôle d'entrée de liquide 100" ou 100"' qui viennent d'être décrits peuvent être intégrés dans une micropompe comme celle qui sera décrite plus loin en relation avec les figures 7 et 8, en tant que valve d'entrée.

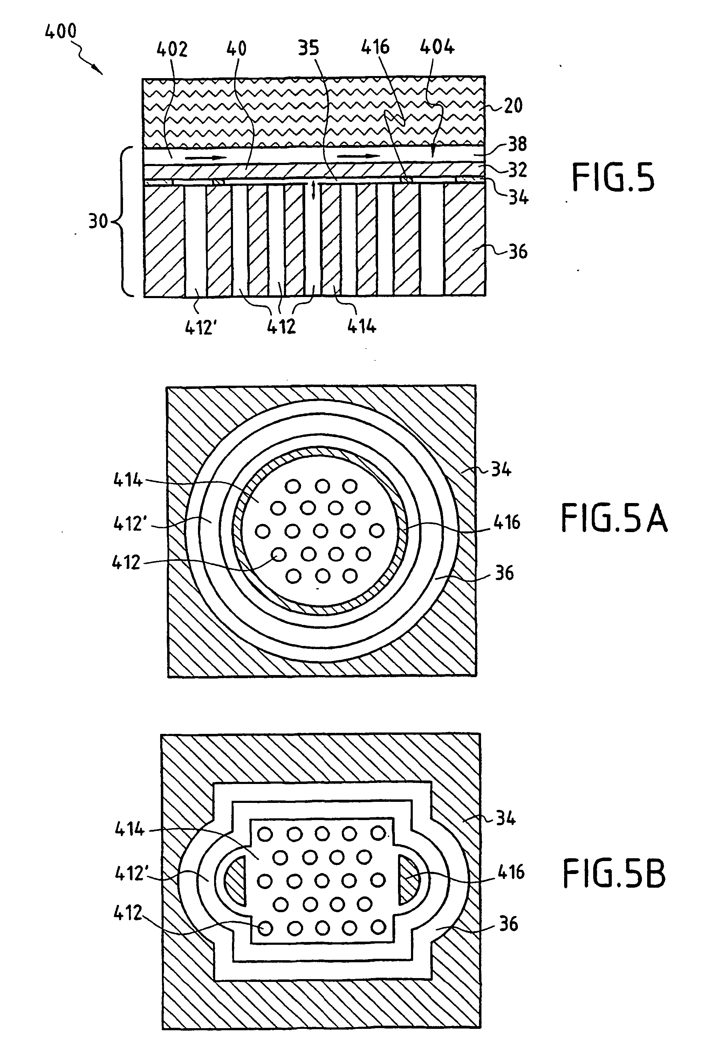

Sur la figure 5 est représenté un deuxième aspect du dispositif fluidique selon la présente invention correspondant à un organe de détection de pression de liquide 400 susceptible de faire partie d'un ensemble fluidique plus complexe pouvant également incorporer l'organe de contrôle d'entrée de liquide 100 présenté préalablement.

Cet organe de détection de pression de liquide 400 comporte une plaque de fermeture de verre 20 disposée au-dessus d'un empilement 30 qui a été préalablement usiné pour la formation des différentes parties fonctionnelles de cet organe de détection de pression de liquide 400.

Cet empilement 30 se compose d'une couche de silicium 32 surmontant une couche d'oxyde de silicium 34, elle-même disposée au-dessus d'une plaque de support en silicium 36.

Ce type d'empilement dénommé communément SOI («SILICON-ON-INSULATOR») est disponible dans le commerce sous la forme de plaques ou "wafer" notamment utilisés dans l'industrie électronique à semiconducteur. Comme pour l'organe de contrôle d'entrée de liquide 100 présenté préalablement, le rôle de ces trois éléments de l'empilement 30 détermine des épaisseurs sensiblement différentes :
- la plaque de support en silicium 36 servant de base rigide, celle-ci présente de préférence une épaisseur située entre 50 et 1000 µm, avantageusement entre 300 et 500 µm,
- la couche d'oxyde de silicium 34 est destinée à relier la plaque de support en silicium 36 et la couche en silicium 32 en maintenant un écart constant entre elles, tout en pouvant être facilement être retirée dans certaines zones de sorte que son épaisseur doit rester très faible, de préférence entre 0,1 à 2 µm et avantageusement entre 0,5 et 1 µm, et
- la couche de silicium 32 est destinée à être usinée dans une partie seulement de son épaisseur (environ la moitié) afin de délimiter, avec la plaque de fermeture de verre 20, une cavité et un organe mobile ;cette couche de silicium 32 , qui peut être réalisée dans du silicium monocristallin ou polycristallin, présente une épaisseur initiale de préférence comprise entre 1 et 100 µm, et avantageusement entre 10 et 50 µm.

L'empilement 30 est usiné par les techniques classiques de photolithographie et attaque chimique afin d'obtenir les différents éléments fonctionnels de de cet organe de détection de pression de liquide 400, notamment une cavité 38 et un organe mobile 40, avant d'effectuer la liaison entre la plaque de fermeture de verre 20 et cet empilement 30. Cette liaison entre la plaque de fermeture 20 et la face libre de la couche de silicium 32 est, de manière connue, effectuée par soudage de plaque (soudage anodique lorsque la plaque de fermeture est en verre) à l'issue duquel on obtient une fixation sous la forme d'une liaison étanche.

Cet organe de détection de pression de liquide 400 comporte la cavité 38 dans laquelle circule un fluide dont le sens d'écoulement a été illustré par deux flèches horizontales sur la figure 5. Sous la pression de ce liquide, l'organe mobile 40 est apte à se mouvoir verticalement en se rapprochant ou en s'éloignant de la plaque de support en silicium 36 (flèche verticale à double pointe) jusqu'à venir en contact avec cette plaque de support en silicium 36.

A titre d'exemple, le parcours du liquide dans la cavité 38 s'effectue donc depuis une entrée 402 située à gauche de la figure 5 jusqu'à une sortie 404 située sur la partie droite de la figure 5

Afin d'effectuer le retrait de matière correspondant à la zone 35 de la couche d'oxyde de silicium 34, une série de conduits 412 de section circulaire est creusée dans toute l'épaisseur de la plaque de support en silicium 36 en regard de cette zone 35 et de l'organe mobile 40.

Comme on peut le voir sur les figures 5A et 5B, ces conduits 412 sont situés à égale distance les uns des autres en regard de toute la zone 35 au niveau de laquelle l'oxyde de silicium est retiré de la couche 34.

Un autre conduit 412' qui présente en section transversale une forme de paroi de cylindre, de préférence annulaire, est creusé dans toute l'épaisseur de la plaque de support en silicium 36 et entoure la série des conduits 412. Ce conduit 412' permet de séparer du reste de la plaque de support en silicium 36 une partie d'appui 414 en forme de cylindre percée par les conduits 412, située en regard de l'organe mobile 40 et connectée à une liaison électrique.

Afin de maintenir la partie d'appui 414 solidaire de l'empilement 30, une portion 416 de la couche d'oxyde de silicium 34 est laissée intacte sur le bord de la partie d'appui 414, à côté du conduit 412'. En effet, cette portion 416 relie la partie d'appui 414 à la couche de silicium 32 en entourant l'organe mobile 40, en formant ainsi des moyens de solidarisation.

Sur la variante de réalisation de la figure 5A, le conduit 412' et la portion 416 présentent une forme de paroi de cylindre de section circulaire tandis que les conduits 412 sont régulièrement répartis sur une zone de forme circulaire.

Dans le cas de la variante de réalisation de la figure 5B, les conduits 412 sont régulièrement répartis sur une zone de forme rectangulaire, la portion 416 est constituée de deux portions en forme de demi cercle situées le long des deux côtés opposés du rectangle précité en formant deux "oreilles". Sur cette figure 5B, le conduit 412' entoure à la fois la zone précitée de forme rectangulaire et les deux portions 416, ce conduit 412' présentant alors une forme de paroi de cylindre de section rectangulaire avec deux oreilles comme une sorte de trèfle à quatre feuilles ou de croix grecque.

Cet organe de détection de pression 400 est conformé de manière que lorsque la pression de liquide dépasse un certain seuil dans la cavité 38, l'organe mobile 40 passe de sa position de repos ou position ouverte (illustrée sur la figure 5) à une position active ou position fermée dans laquelle l'organe mobile 40 vient en contact avec la partie d'appui 414 de la plaque de support en silicium 36.

Dans ce cas, le contact entre la couche 32 (à l'emplacement de l'organe mobile 40) et la plaque 36 (à l'emplacement de la partie d'appui 414), toutes deux réalisées dans un silicium dopé formant un semiconducteur jouant le rôle d'un conducteur électrique intégré dans un circuit capacitif, crée, entre les liaisons électriques respectivement connectées à la couche 32 et à la partie d'appui 414 de la plaque de support 36, une augmentation brutale de capacité dont la détection permet de savoir qu'un niveau de pression liquide prédéterminé a été atteint dans la cavité 38.

D'autres variantes de réalisation sont envisageables, notamment celle de créer deux électrodes, formant deux parties d'appui, séparées l'une de l'autre et du reste de la plaque 36.

Cet organe de détection de pression de liquide 400 forme un capteur de pression de liquide qui fonctionne de manière capacitive. Toutefois, d'autres types de capteur peuvent être créés grâce à cet organe 400: un capteur à effet tunnel, à contact Schotky, par détection inductive, par détection optique (par exemple en utilisant une diode laser qui repère la courbure de l'organe mobile 40) ou utilisant une jauge de contrainte.

Un tel organe de détection de pression de liquide 400 est très utile au niveau d'un ensemble fluidique car il permet de repérer, en fonction du niveau de pression qui déclenche le contact entre l'organe mobile 40 et la partie d'appui 414, qu'un niveau de pression prédéterminé est atteint dans la cavité 38.

II est entendu que ce détecteur de pression 400 est un capteur différentiel prenant pour référence de pression la pression extérieure régnant dans les conduits 412 et 412' et dans la zone 35 exempte de matière.

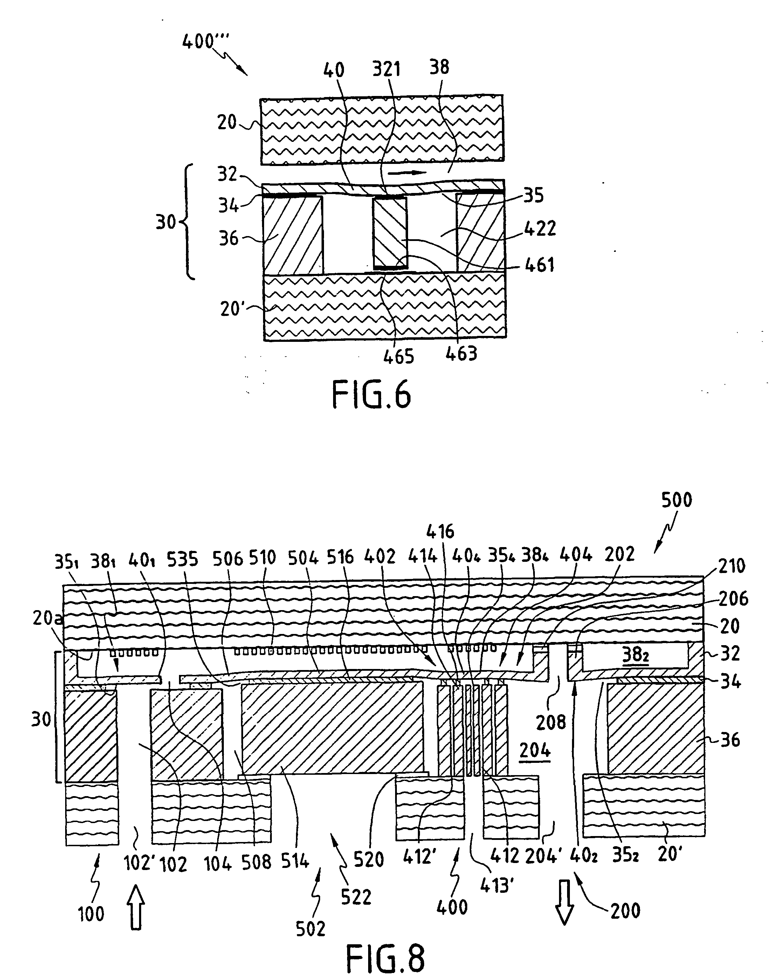

La figure 6 concerne un deuxième mode de réalisation de l'organe de détection de pression de liquide constituant le deuxième aspect de l'invention. Dans ce cas, l'organe de détection de pression de liquide 400"' comporte en outre, par rapport aux premier mode de réalisation de l'organe de détection de pression de liquide des figures 5, 5A et 5B, une deuxième plaque de verre 20'.

Ainsi, dans cet organe de détection de pression de liquide 400"', la plaque de fermeture supérieure 20 est une première plaque de fermeture 20 en verre et la deuxième plaque de verre 20' forme une deuxième plaque de fermeture qui est fixée sur la face de la plaque de support 36 opposée à ladite première plaque de fermeture 20 en verre.

La plaque de support 36 est munie d'un conduit 422 la traversant de part en part.

Pour déporter la zone de contact électrique engendrant la détection du seuil de pression vers la deuxième plaque de fermeture 20' en verre, dans le deuxième mode de réalisation de l'organe de détection de pression de liquide 400"', la partie qui forme un îlot séparé du reste de la plaque de support 36 constitue une partie mobile 461.

A cet effet, le conduit 422 est annulaire afin de séparer la partie mobile 461 du reste de la plaque de support 36 et la couche de matériau isolant 34 présente une zone de liaison 321 entourée par la zone 35 et reliant de manière solidaire ladite partie mobile 461 audit organe mobile 40.

Afin de remplir les fonctions de détecteur de pression, cet organe de détection de pression de liquide 400"' comporte en outre un premier élément conducteur 463 situé sur la face de ladite partie mobile 461 placée en regard de la deuxième plaque de fermeture 20' en verre et un deuxième élément conducteur 465 situé sur la face de la deuxième plaque de fermeture 20' en verre placée en regard de ladite partie mobile 461. Bien entendu, lesdits premier et deuxième éléments conducteurs 463, 465 sont aptes à former un contact électrique lorsque ledit organe mobile 40 et ladite partie mobile 461 qui lui est solidaire, se rapprochent de la deuxième plaque de fermeture 20' en verre.

Alternativement à ce contact électrique, d'autres méthode de détection peuvent être utilisées : mesure capacitive, inductive, optique ou par des jauges de contraintes disposées sur l'organe mobile 40. Dans ces autres cas la présence et/ou l'emplacement du premier élément conducteur 463 et/ou du deuxième élément conducteur 465 sera à adapter à la technique de détection employée.

Comme pour le détecteur de pression 400 décrit en relation avec les figures 5, 5A et 5B, le détecteur de pression de liquide 400"' permet de repérer, par la déformation qui va en augmentant de l'organe mobile 40 du fait de l'augmentation de la pression de liquide dans la chambre de pompage, un seuil de pression déterminé, par rapport à le pression extérieure.

Ainsi, le deuxième mode de réalisation de l'organe de détection de pression, illustré sur la figure 6, appartient à un deuxième type de dispositif fluidique dans lequel une deuxième plaque de verre 20' est nécessaire pour déporter le contact électrique entre cette deuxième plaque de verre 20' et une partie mobile 461 de la plaque de support 36 de l'empilement 30.

Les organes de contrôle de détection de pression 400 et 400'" qui viennent d'être décrits peuvent être intégrés dans une micropompe comme celle qui sera décrite plus loin en relation avec les figures 7 et 8, en tant que valve d'entrée.

Pour ce qui est du procédé de fabrication de l'organe de contrôle de détection de pression 400"', il suffit, par rapport au procédé de fabrication de l'organe de contrôle de détection de pression 400 selon le premier mode de réalisation (figures 5, 5A, 5B), de prévoir une deuxième plaque de fermeture 20' sur laquelle on aura déposé un deuxième élément conducteur 465 réalisé dans un matériau conducteur de l'électricité et de la relier à la plaque de support en silicium 36. Ces deux étapes seront réalisées à la fin du procédé de fabrication, c'est à dire après traitement (notamment par usinage et/ou structuration) de l'empilement 30 et après avoir muni la face de la partie mobile 463 tournée en direction opposée à l'organe mobile 40 d'un premier éléments conducteur 463 réalisé dans un matériau conducteur de l'électricité. Il faudra bien entendu prévoir de relier électriquement les premier et deuxième éléments conducteurs 463, 465 au circuit du système de détection.

II est à noter que le terme "conduit" employé pour les canaux ou volumes 412, 412' et 422 des deux modes de réalisation 400 et 400"' du détecteur de pression ne sont pas destinés au passage de liquide pendant le fonctionnement de cet organe fluidique.

Les premier et deuxième aspects du dispositif fluidique selon la présente invention qui viennent d'être décrits en relation avec les figures 1 à 6 réalisent des fonctions différentes en application à un passage de liquide dans un dispositif fluidique et présentent une structure simple analogue pouvant être réalisée selon un procédé de fabrication très simple à mettre en oeuvre.

En outre, ce procédé de fabrication des différents organes 100, 100", 100"', 400 et 400"' présentés précédemment présente de grandes similitudes de sorte que ces différents organes fluidiques 100, 100", 100"', 400 et 400"' peuvent facilement être disposés dans un même ensemble fluidique.

Un tel exemple d'intégration sera illustré ci-après en relation avec les figures 7 à 15. Les étapes communes du procédé de fabrication des organes 100, 100", 100"', 400 et 400"' ont été énoncées au début de la description en relation avec un procédé de fabrication d'un dispositif fluidique, lequel procédé présente des adaptations pour la réalisation de chacun des organes 100, 100", 100"', 400 et 400"' comme énoncé ci-après.

Le procédé de fabrication de l'organe de contrôle d'entrée de liquide 100 tel qu'illustré sur la figure 1 comprend les étapes suivantes :
a) on fournit un empilement 30 comprenant une plaque de support 36, de préférence en silicium, une couche d'oxyde de silicium 34 couvrant, au moins partiellement, la plaque de support 36, et une couche de silicium 32 (monocristallin ou polycristallin) recouvrant la couche d'oxyde de silicium 34 et présentant une face libre opposée à une face recouvrant ladite couche d'oxyde de silicium 34,
b) on usine la cavité 38 à partir de la face libre de la couche de silicium 32 par photolithographie et attaque chimique,
c) on usine le dégagement 104 à partir de la face libre de la couche de silicium 32 par photolithographie et attaque chimique sur toute l'épaisseur de la couche de silicium 32 jusqu'à atteindre la couche d'oxyde de silicium 34,
d) on usine par photolithographie et attaque chimique, depuis l'autre côté de l'empilement 30, le conduit d'entrée de liquide 102 traversant de part en part la plaque de support 36,
e) on réalise, à travers ce conduit 102 et le dégagement 104, une attaque chimique de la couche d'oxyde de silicium 34 de façon à créer la zone 35 exempte de matière dans la couche d'oxyde de silicium 34 de sorte que la zone de la couche de silicium 32 située en regard de cette zone 35 se trouve libérée de la couche d'oxyde de silicium 34 en formant ainsi l'organe mobile 40, lequel reste relié à la couche de silicium 32 par le ou les bras 41,
f) on fournit la plaque de fermeture 20, et
g) on relie de façon étanche, par des moyens physico-chimiques, la plaque de fermeture 20 à la surface de la couche de silicium 32 qui n'a pas été usinée, de préférence par soudage de plaque ("wafer bonding").

Lorsque la plaque de fermeture 20 est en verre la technique précitée de soudage de plaque consiste en un soudage anodique. Si la plaque de fermeture est en silicium, un soudage direct permettra la liaison étanche avec la couche de silicium 32.

Ainsi, on comprend que le traitement par micro-usinage de l'empilement 30 s'effectue de manière indépendante pour chacune de ses faces de sorte que les groupes d'étapes b) et c), d'une part, et d) et e), d'autre part peuvent être réalisées l'une avant l'autre comme décrit précédemment ou l'une après l'autre.

L'organe de détection de pression de liquide 400 illustré à la figure 5 et représentant le deuxième aspect de la présente invention est fabriqué selon un procédé qui comporte les étapes suivantes :
a) on fournit un empilement 30 comprenant une plaque de support 36, de préférence en silicium, une couche d'oxyde de silicium 34 recouvrant, au moins partiellement, la plaque de support 36, et une couche de silicium 32 (monocristallin ou polycristallin) recouvrant la couche 34 et présentant une face libre opposée à une face recouvrant la couche d'oxyde de silicium 34,
b) on usine la cavité 38 à partir de la face libre de la couche de silicium 32 par photolithographie et attaque chimique,
c) on usine, depuis l'autre côté de l'empilement 30, les conduits 412 et 412' traversant de part en part la plaque de support 36 depuis l'autre côté de l'empilement 30,
d) on réalise une attaque chimique de la couche d'oxyde de silicium 34 par les conduits 412 et 412' afin de former la zone 35 de la couche d'oxyde de silicium 34 exempte de matière, toute en laissant de l'oxyde de silicium au niveau de la portion 416, et afin de libérer l'organe mobile 40 de la couche d'oxyde de silicium 34,
e) on fournit la plaque de fermeture 20, et
f) on relie de façon étanche par un procédé physico-chimique la plaque de fermeture 20 à la surface de la couche de silicium 32 qui n'a pas été usinée, de préférence par soudage de plaque ("wafer bonding").

Que ce soit pour l'un des organes de contrôle d'entrée de liquide 100, 100", 100"' ou pour l'un des organes de détection de pression de liquide 400, 400"', on comprend que l'usinage de la cavité 38, située entre la couche de silicium 32 et la plaque de fermeture 20, puisse se faire également par usinage de la couche 32 et de la plaque 20 ou par usinage de la plaque 20 seulement.

On se reportera maintenant aux figures 7 à 9 illustrant une micropompe 500 formant un ensemble fluidique intégrant un organe de contrôle d'entrée de liquide 100, une partie de pompage 502, un organe de détection de pression 400 et un organe de contrôle de sortie de liquide 200.

De manière préférentielle, outre la plaque de fermeture en verre 20 et l'empilement 30, la micropompe est munie d'une plaque de fermeture en verre supplémentaire 20' fixée par soudage de plaque sur la face de la plaque de support 36 opposée à la plaque de fermeture de verre 20, c'est-à-dire en partie basse sur les figures 7 et 8.

Ainsi, on comprend que la plaque de fermeture 20 constitue une première plaque de fermeture en verre et que la plaque de fermeture supplémentaire 20' constitue une deuxième plaque de fermeture en verre fixée sur la face de la plaque de support 36 opposée à la première plaque de fermeture en verre 20.

Comme il sera détaillé plus loin, la plaque de fermeture en verre 20 sert, en plus de refermer de manière étanche l'espace de la micropompe rempli de liquide, de butée lors du mouvement ascendant de la membrane de pompage 506. Afin d'empêcher une adhésion ou un effet de ventouse entre la membrane de pompage et la plaque de fermeture 20, des éléments 510 réalisés dans un matériau anti-adhésion sont situés sur la face 20a de la plaque de fermeture 20 tournée en direction de l'empilement.

De préférence, ces éléments 510 sont issus d'une couche en titane déposée sur la face 20a précitée de la plaque de fermeture 20. Ces éléments 510 forment des saillies séparées les unes des autres qui permettent un écoulement de liquide entre elles tout en empêchant à la membrane de pompage 506 d'adhérer à la plaque de fermeture 20.

II est à noter que ces éléments 510 peuvent également être situés sur la couche de silicium 32, à savoir notamment sur la face libre de la membrane 506.

La plaque de fermeture 20' sert elle aussi d'élément de butée dans le mouvement descendant de membrane 506 par contact entre la plaque 20' et la partie mobile de pompage 514. La combinaison de ces deux butées (plaques 20 et 20') permet de contrôler l'amplitude du mouvement vertical de la membrane 506 et d'assurer la précision du volume pompé.

Afin de garder cette partie 514 mobile, est prévue (voir figures 7 et 8) une couche anti-adhésion 520 sur la face de la plaque de fermeture supplémentaire 20' tournée vers l'empilement 30. Cette couche 520 est en forme d'anneau et positionnée sur le bord d'une ouverture 522 traversant la plaque de fermeture supplémentaire 20'.

La couche 520 est de préférence réalisée en titane et empêche ainsi la fixation entre la partie mobile de pompage 514 et la plaque de fermeture supplémentaire 20' lors du soudage de plaque entre l'empilement 30 et la plaque de fermeture supplémentaire 20'.

Bien entendu, la couche 520 aurait pu être déposée sur la face de la partie mobile de pompage 514 tournée en direction opposée à l'empilement 30.

Pour les éléments 110, 510 ou 520, le titane peut avantageusement être remplacé par un autre matériau anti-adhésion comme l'or, l'oxyde de silicium ou le nitrure de silicium.

Dans la partie amont de la micropompe 500, on retrouve l'organe de contrôle de l'entrée de liquide 100, le conduit d'entrée de liquide 102 étant prolongé au niveau de la plaque de fermeture supplémentaire de verre 20' par un conduit d'entrée de liquide 102' à l'entrée duquel arrive le liquide destiné à être fourni via la micropompe 500.

Cet organe de contrôle d'entrée de liquide 100 comprend une zone 35₁ de la couche d'oxyde de silicium 34 exempte de matière, la cavité 38₁ et le dégagement 104 qui délimitent l'organe mobile 40₁. L'organe de contrôle d'entrée de liquide 100 est illustré en position de repos sur les figures 7 et 8.

Entre l'organe de contrôle d'entrée de liquide 100 et un détecteur de pression 400, la micropompe 500 comprend la partie de pompage 502 munie d'une chambre de pompage 504 située dans le prolongement de la cavité 38₁ et délimitée entre la plaque de fermeture de verre 20 et la couche de silicium 32 qui a été usinée sur sa face tournée vers la plaque de fermeture de verre 20.

Une membrane de pompage 506 en forme de disque est située dans la couche de silicium 32, en regard d'une part de la chambre de pompage 504 et d'autre part d'un volume annulaire exempt de matière 508 usiné dans la plaque de support 36, ce volume annulaire exempt de matière 508 étant prolongé, dans la couche d'oxyde de silicium 34 au niveau d'une zone exempte de matière 535.

Ce volume 508 permet de séparer du reste de la plaque de support en silicium 36 une partie mobile de pompage 514 en forme de cylindre plein et de section circulaire, située en regard de la membrane de pompage 506 à laquelle elle est reliée par une portion 516 de la couche d'oxyde de silicium 34 laissée intacte.

Le volume 508 étant isolé du reste de la plaque de support 36, on peut avantageusement intégrer dans la partie mobile de pompage 514 au moins un organe de détection de pression de liquide, par exemple sous la forme d'un ou plusieurs détecteurs de pression de liquide fonctionnant de manière similaire à celui des figures 5, 5A et 5B.

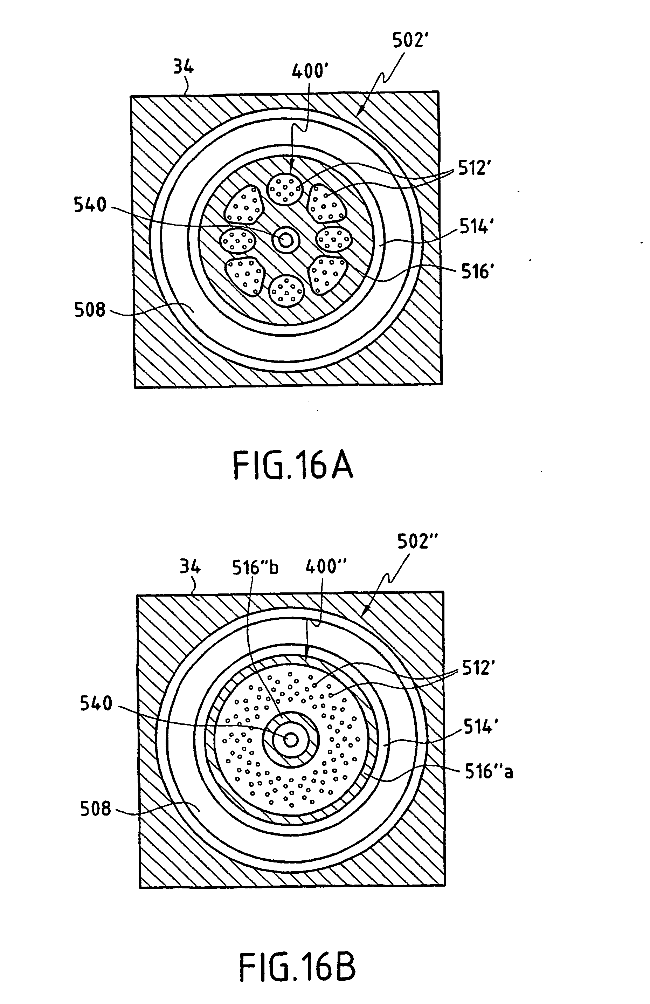

Une telle variante de réalisation est illustrée sur la figure 16A qui illustre une partie de pompage 502' munie de huit détecteurs de pression de liquide 400' régulièrement angulairement répartis au niveau d'une partie mobile de pompage 514' percée de part en part de huit séries de conduits 512'. Ces huit séries de conduits 512' sont isolées les unes des autres par une portion 516' de la couche d'oxyde de silicium 34 laissée intacte sauf, pour chaque détecteur 400', dans une zone regroupant la série correspondante de conduits 512'.

Il est entendu que l'on peut prévoir une partie de pompage 502' qui est munie d'au moins deux détecteurs de pression de liquide 400' formant chacun un organe de détection de pression de liquide et qui sont régulièrement angulairement répartis au niveau de ladite partie mobile de pompage 514' qui est percée de part en part d'au moins deux séries de conduits 512'.

Une autre variante de réalisation de la partie de pompage est illustrée sur la figure 16B sous la référence numérique 502". Dans ce cas, les huit séries de conduits 512' de la figure 16A ont été complétées par d'autres conduits 512' afin que tous ces conduits recouvrent une zone annulaire destinée à former un détecteur de pression de liquide annulaire 400". Pour cette autre variante de réalisation, la portion de la couche d'oxyde de silicium 34 laissée intacte se limite à une première portion annulaire 516"a située sensiblement au bord de la partie mobile de pompage 514' et à une deuxième portion 516"b située essentiellement au centre de la partie mobile de pompage 514'.

La partie mobile de pompage 514' est également percée, de préférence en son centre, de part en part par un passage 540 dans lequel est susceptible d'être logée une tige de commande (non représentée) dont une extrémité est fixée sur à la membrane 506 et dont l'autre extrémité, faisant saillie hors de l'ouverture 522, forme une poignée. Cette poignée permet à un utilisateur, en cas de nécessité, de tirer, au moyen de la tige précitée, sur la membrane 506 afin de l'écarter de la plaque de fermeture 20. Une telle action peut être mise en oeuvre en série pour développer des dépressions d'aspiration importantes successives dans la chambre de pompage 504 ou bien pour accélérer le fonctionnement de la micropompe. II est à relever que la présence du passage 540 et de la tige est indépendante de la présence d'un organe de détection de pression dans la partie mobile de pompage 514.

II est à noter que les moyens de commande de la micropompe, situés en regard de la membrane de pompage 506 et dénommés de manière générique actionneur, peuvent être intégrés directement à la micropompe, par fixation sur la face de la plaque de verre 20' tournée en direction opposée à l'empilement et par fixation sur la partie mobile de pompage 514, ou être externes à la micropompe, en étant reliés indirectement à la membrane de pompage 506.

Ces moyens de commande peuvent être notamment à fonctionnement de type piézo-électrique, électromagnétique ou pneumatique.

En aval de la partie de pompage 502, la micropompe 500 illustrée sur les figures 7 et 8 comprend le détecteur de pression de liquide 400 décrit en relation avec les figures 5,5A et 5B, le passage 412 étant relié à la pression extérieure par le biais d'un conduit 413' traversant la plaque de fermeture de verre supplémentaire 20'. En outre, on retrouve les autres éléments constitutifs de l'organe de détection de pression de liquide 400, à savoir la zone 35₄ de la couche d'oxyde de silicium 34 exempte de matière, la cavité 38₄ qui délimite l'organe mobile 40₄ entre une entrée de liquide 402 et une sortie de liquide 404.

Sur les figures 7 et 8, l'organe de détection de pression de liquide 400 est représenté en position de repos ou position ouverte, c'est-à-dire avec un organe mobile 40₄ qui n'est pas en contact avec la partie d'appui 414. Il est à noter que les liaisons électriques, de la partie d'appui 414 d'une part et de la couche de silicium 32 d'autre part, ne sont pas représentées.

Ainsi, on comprend que le détecteur de pression sert à vérifier le bon fonctionnement de la micropompe par la détection de l'augmentation transitoire de pression de liquide à chaque coup de pompage qui résulte de la déflexion de la membrane de pompage 506 (une augmentation de la pression correspondant à un mouvement vers le haut de la membrane 506 sur les figures 7 et 8 et inversement). On peut détecter soit l'absence de pompage par l'absence d'augmentation de pression ou la présence d'une occlusion en aval par la durée exagérément longue de la surpression.

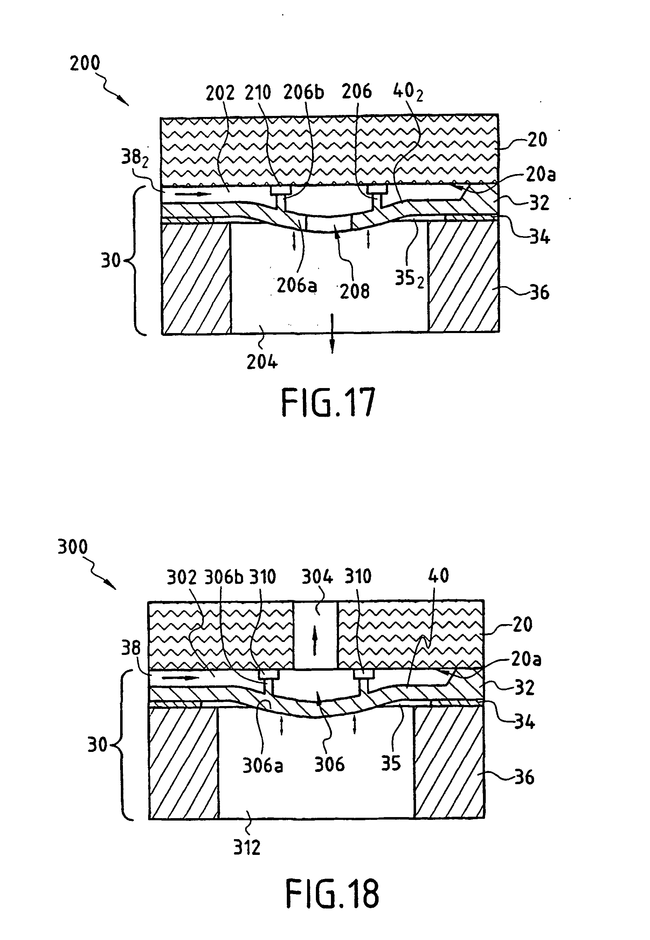

Dans la partie la plus aval de la micropompe 500, se trouve un organe de contrôle de sortie de liquide 200 illustré de manière agrandie sur la figure 17.

Grâce à cet organe de contrôle de sortie de liquide 200, qui forme une valve anti-retour, la sortie de liquide s'effectue par un conduit de sortie de liquide 204 de la plaque de support en silicium 36 qui est prolongé, au niveau de la plaque de fermeture de verre supplémentaire 20', par un conduit de sortie de liquide 204'.

Les autres éléments constitutifs de cet organe de contrôle de sortie de liquide 200 sont une zone 35₂ exempte de matière de la couche d'oxyde de silicium 34, un organe mobile 40₂ délimité par la cavité 38₂, cet organe mobile 40₂ comprenant une portion annulaire 206 formant le corps de la valve et dont la deuxième extrémité vient en contact sur une couche anti-adhésive 210 située sur la plaque de fermeture 20, la portion annulaire 206 étant traversée par un orifice 208. L'organe de contrôle de sortie 200 est illustré sur les figures 7 et 8 en position fermée.

L'organe de contrôle de sortie de liquide 200 illustré de manière agrandie sur la figure 17, et sa variante de réalisation 300 illustrée sur la figure 18, sont réalisés à partir des mêmes éléments que l'organe 100 de la figure 1.

Comme on peut le voir sur la figure 17, l'organe de contrôle de sortie de liquide 200 présente une entrée de liquide 202 au niveau de la cavité 38₂ et un conduit de sortie de liquide 204 usiné dans toute l'épaisseur de la plaque de support en silicium 36, en regard de la cavité 38.

La zone 35₂ de la couche d'oxyde de silicium 34 qui est exempte de matière se trouve au moins dans le prolongement du conduit de sortie de liquide 204 et s'étend légèrement au-delà tout autour de ce conduit 204.

Lors de la réalisation de la cavité 38 par usinage de la couche de silicium 32, l'organe mobile 40 a été formé avec une portion 206 s'étendant dans sensiblement toute l'épaisseur initiale de la couche de silicium 32 et présentant un contour fermé, de préférence annulaire. Cette portion 206 s'étend depuis une première extrémité 206a faisant face à la zone 35₂ de la couche de matériau isolant 34 jusqu'à une deuxième extrémité 206b proche de la face 20a de la plaque de fermeture 20 tournée vers l'empilement 30.

Cette portion 206 présente une forme de manchon cylindrique, de préférence annulaire, et entoure un orifice 208 situé dans le prolongement de la zone 35₂ et du conduit 204 avec lequel l'orifice 208 est en communication de fluide.

Dans cet organe de contrôle de sortie de liquide 200, l'organe mobile 40 s'étend sensiblement dans le prolongement de toute la section du conduit de sortie de liquide 204.

Le siège de la valve est constitué par un élément anti-adhésion 210, de préférence en titane, situé sur la face 20a de la plaque de fermeture de verre 20 placée en regard de l'organe mobile 40₂. Cet élément anti-adhésion 210 présente une forme similaire à celle de la portion 206, donc de préférence annulaire. Cet élément anti-adhésion 210 pourrait aussi être situé sur la deuxième extrémité 206b de la portion 206 et être réalisé également en un autre matériau anti-adhésion comme l'or, l'oxyde de silicium ou le nitrure de silicium.

Le corps de la valve est constitué par la deuxième extrémité 206b de la portion annulaire 206 dont la première extrémité 206a est tournée en direction de la plaque de support en silicium 36 et de manière adjacente au conduit de sortie de liquide 204.

Afin de minimiser les surfaces en contact de la valve, la deuxième extrémité 206b de la portion annulaire 206 présente une épaisseur réduite, l'orifice 208 étant plus large à ce niveau.

Sur la figure 17, l'organe de contrôle de sortie de liquide 200 est illustré en position de repos correspondant à une position fermée, le liquide arrivant par l'entrée 202 étant empêché de pénétrer dans l'orifice 208 par la portion 206 dont la deuxième extrémité 206b est en contact étanche avec ledit élément anti-adhésion 210.

Une pression de liquide suffisante dans l'entrée de liquide 202 va exercer une force sur l'organe mobile qui va permettre, si la pression de liquide dans le conduit de sortie 204 est inférieure à la pression d'entrée de liquide, l'ouverture de la valve par rapprochement de l'organe mobile 40₂ en direction de la plaque de support en silicium 36 (cas de figure non représentée). Dans cette position ouverte, le liquide est apte à franchir la deuxième extrémité 206b de la portion 206 qui s'est écartée dudit élément anti-adhésion 210 et de la plaque de fermeture 20, pour pénétrer dans l'orifice 208 qui est directement en communication de fluide avec le conduit de sortie de liquide 204.

On comprend également que ledit élément anti-adhésion 210 permet d'éviter la solidarisation entre le corps de la valve formé de la deuxième extrémité 206b de la portion 206 contre le siège de la valve (face dudit élément anti-adhésion 210 tournée vers l'empilement 30).

En outre, on comprend que ledit élément anti-adhésion 210 permet, par un déplacement élastique initial de l'organe mobile 40₂, de créer une prétension dans l'organe de contrôle de sortie de liquide 200, à savoir que la valve reste fermée dans sa position de repos et pour une pression de liquide ne dépassant pas un seuil prédéterminé.

C'est également par un phénomène de retour élastique que, lorsque la pression de liquide dans l'entrée 202 est inférieure ou égale à la pression de liquide dans le conduit de sortie 204, l'organe de contrôle 200 revient dans sa position fermée illustrée sur la figure 17, le contact étanche entre la deuxième extrémité 206b de la portion 206 et ledit élément anti-adhésion 210 empêchant tout passage ultérieur de liquide depuis l'entrée de liquide 202 jusqu'à l'orifice 208.

Sur la figure 18 est représentée une variante de réalisation correspondant à un organe de contrôle de sortie de liquide 300 pour lequel l'entrée de liquide 302 est réalisée dans la cavité 38 tandis que le conduit de sortie de liquide 304 traverse de part en part la plaque de fermeture de verre 20.

L'organe mobile 40 de cet organe de contrôle de sortie de liquide 300 présente une forme très similaire à l'organe mobile 40 de la figure 17: il comporte une portion annulaire 306 similaire à la portion 206 mais ne comprend toutefois pas d'orifice tel que l'orifice 208.

En effet, dans ce cas, la portion annulaire 306 joue toujours le rôle de corps de valve par un contact étanche (position fermée telle qu'illustrée sur la figure 18) entre la deuxième extrémité 306b de la portion annulaire 306 tournée en direction de la plaque de fermeture et face d'un élément anti-adhésion 310 tournée vers l'empilement 30.

Dans ce cas de figure, afin de permettre le débattement vertical de l'organe mobile 40, schématisé par la flèche à double pointe sur la figure 18, la zone 35 de la couche d'oxyde de silicium 34 exempte de matière s'étend en regard de tout l'organe mobile 40.

En outre, un accès à la couche d'oxyde de silicium 34 est réalisé depuis la face libre de la plaque de support en silicium 36, pour le retrait de l'oxyde de silicium dans la zone 35, au moyen d'un passage 312 traversant de part en part la plaque de support en silicium 36. Ce passage 312 présente de manière préférentielle mais non limitative une forme de cylindre avec une forme circulaire en section transversale telle qu'illustrée sur la figure 18.

La micropompe 500 peut être utilisée dans de nombreuses applications, notamment comme pompe à usage médical pour la délivrance en continu d'un médicament liquide.

Une telle pompe peut être, grâce à ses dimensions très réduites, de type « implantable », c'est-à-dire pouvant être disposée sous la peau du patient, ou bien de type « exteme » et reliée, par son organe de contrôle d'entrée 100, au système circulatoire sanguin du patient par un port d'entrée au niveau de la peau.

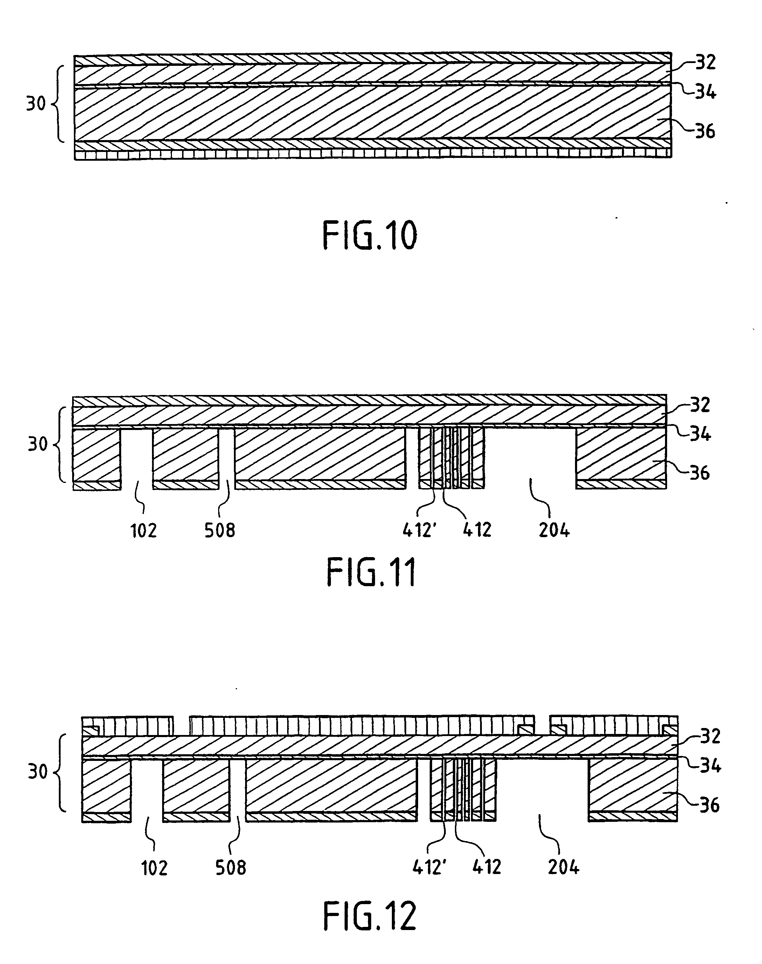

Sur les figures 10 à 15 sont illustrées les différentes étapes de fabrication de la micropompe 500 et qui comprennent notamment les étapes de fabrication des organes 100, 200 et 400 et de la partie de pompage 502, ces étapes étant réalisées de manière simultanée.

Parmi ces différentes étapes de fabrication, on distingue le terme « usiner », réservé à un usinage, destiné à faire varier l'épaisseur d'une plaque ou de certaines zones d'une plaque, du terme « structurer », réservé à un usinage qui privilégie la conservation de la matière d'une couche dans certaines zones et le retrait de toute la matière de cette couche dans d'autres zones.

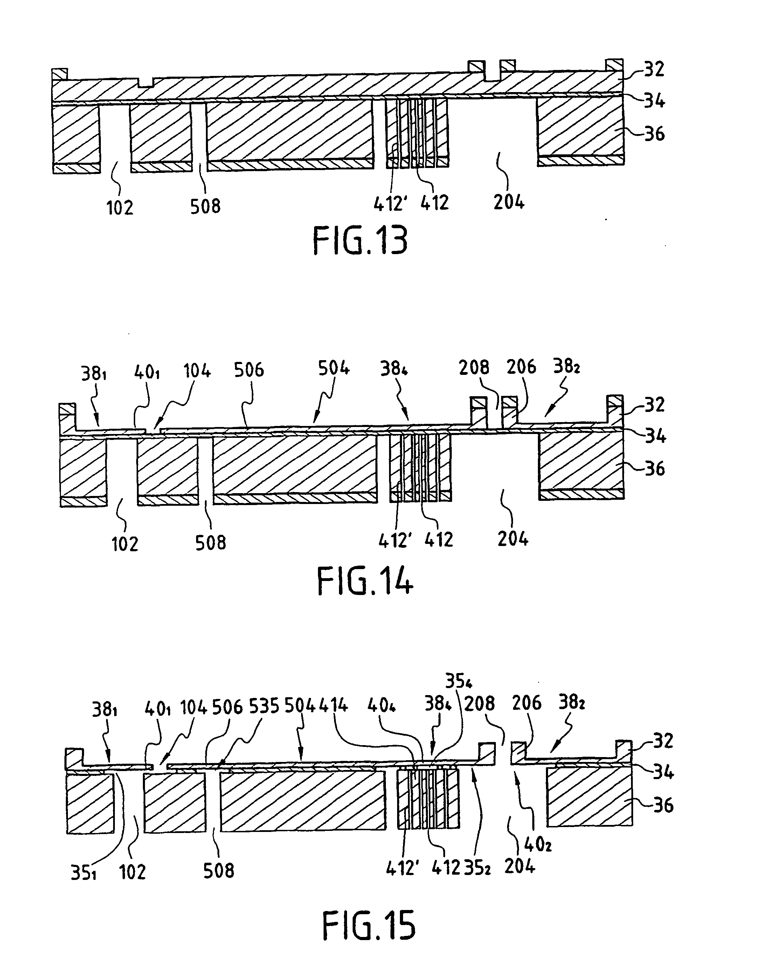

Le procédé de fabrication de la micropompe 500 comporte en effet les étapes suivantes :
a) on fournit un empilement 30 comprenant une plaque de support 36, de préférence en silicium, une couche de matériau isolant 34, de préférence en oxyde de silicium, recouvrant au moins partiellement la plaque de support 36, et une couche de silicium 32 (monocristallin ou polycristallin) recouvrant la couche de matériau isolant 34 et présentant une face libre opposée à une face recouvrant la couche de matériau isolant 34,
b) on usine, par photolithographie et attaque chimique depuis la face libre de la plaque de support 36, le conduit d'entrée de liquide 102 de l'organe de contrôle d'entrée 100, le volume annulaire 508, les conduits 412 et 412' du détecteur de pression 400, et le conduit de sortie de liquide 204 de l'organe de contrôle de sortie de liquide 200, ces conduits ou volume annulaire traversant de part en part la plaque de support 36 (figure 11),
c) on usine, par photolithographie et attaque chimique (acide fluorhydrique ou BHF) depuis l'autre côté de l'empilement 30, c'est-à-dire à partir de la face libre de la couche de silicium 32, le dégagement 104 et la cavité 38₁ de l'organe de contrôle d'entrée 100, la chambre de pompage 504, la cavité 38₄ du détecteur de pression 400, la cavité 38₂ et l'orifice 208 de l'organe de contrôle de sortie de liquide 200 (figures 9 à 14),
d) on réalise une attaque chimique de la couche d'oxyde de silicium 34 par le conduit d'entrée de liquide 102 de l'organe de contrôle d'entrée 100, par le volume annulaire 508, les conduits 412 et 412' du détecteur de pression 400, et par le conduit de sortie de liquide 204 de l'organe de contrôle de sortie de liquide 200 afin de former les zone 35₁, 535, 35₄ et 35₂ de la couche d'oxyde de silicium 34 exemptes de matière qui permettent respectivement de libérer, de la couche d'oxyde de silicium 34, l'organe mobile 40₁, la membrane 506, l'organe mobile 40₄, et l'organe mobile 40₂ (figure 15),
e) on fournit la première plaque de fermeture 20,
f) on dépose par un procédé physico-chimique une couche d'un matériau anti-adhésion, de préférence du titane, sur une face 20a de la première plaque de fermeture 20 destinée à être reliée audit empilement 30,
g) on structure la couche de matériau anti-adhésion afin de former lesdits éléments 510 et ladite couche anti-adhésion 210,
h) on fournit la deuxième plaque de fermeture 20',
i) on dépose par un procédé physico-chimique une couche d'un matériau anti-adhésion, de préférence du titane, sur une face de la deuxième plaque de fermeture 20 destinée à être reliée audit empilement 30,
j) on structure la couche de matériau anti-adhésion afin de former ladite couche 520 en forme d'anneau,
k) on relie de façon étanche par un procédé physico-chimique la première plaque de fermeture 20 à la surface de la couche de silicium 32 qui n'a pas été usinée, de préférence par soudage de plaque ("wafer bonding"), et
l) on relie de façon étanche par un procédé physico-chimique la deuxième plaque de fermeture 20' à la surface de la plaque de support 36 qui n'a pas été usinée, de préférence par soudage de plaque ("wafer bonding").

On comprend que la micropompe 500 ainsi obtenue est fabriquée de manière très simple et présente des caractéristiques d'épaisseur de chacune de ces parties constitutives très régulières du fait qu'elle est réalisée à partir de l'empilement 30 initial, ce qui assure notamment un volume mort de pompage très limité.

A titre d'illustration de la simplification du procédé de fabrication, pour réaliser une micropompe de l'art antérieur un nombre de masques photolithographiques de l'ordre de 12 était nécessaire pour réaliser et usiner toutes les différentes couches alors qu'avec le procédé précité selon la présente invention, un nombre de masques de l'ordre de 5 est suffisant.

## Revendications

1. Procédé de fabrication d'un dispositif fluidique, **caractérisé en ce qu'**il comporte les étapes suivantes :
- on fournit un empilement (30) comprenant une plaque de support (36), une unique couche de matériau isolant (34) recouvrant au moins partiellement ladite plaque de support (36), et une couche de silicium (32) monocristallin ou polycristallin recouvrant ladite couche de matériau isolant (34) et présentant une face libre,
- on fournit au moins une plaque de fermeture (20),
- on usine une cavité (38) à partir de ladite plaque de fermeture (20) et/ou de la face libre de ladite couche de silicium (32) par photolithographie et attaque chimique,
- on usine au moins un conduit (102; 412, 412') traversant de part en part ladite plaque de support (36) par photolithographie et attaque chimique.
- on réalise une attaque chimique de ladite couche de matériau isolant (34) au moins par ledit conduit (102 ; 412, 412') de sorte qu'une zone de ladite couche de silicium (32) est libérée de ladite couche de matériau isolant (34) en formant un organe mobile (40) adjacent à ladite cavité et apte, sous une pression de liquide dans ladite cavité (38), à se rapprocher, de manière réversible, de ladite plaque de support (36),
- on relie de façon étanche par un procédé physico-chimique, de préférence par soudage de plaque, ladite plaque de fermeture (20) à ladite face libre de la couche de silicium (32).

2. Dispositif fluidique (100 ; 400 ; 500) comprenant un empilement (30) de type SIUCON-ON-INSULATOR « SOI » recouvert d'une plaque de fermeture (20), ledit empilement (30) comportant une plaque de support (36), une unique couche de matériau isolant (34) recouvrant au moins Partiellement ladite plaque de support (36), et une couche de silicium (32) monocristallin ou palycristallin recouvrant ladite couche de matériau isolant (34) et recouverte de ladite plaque de fermeture (20),
ladite plaque de fermeture (20) et/ou ladite couche de silicium (32) étant usinée(s) afin de définir, entre ladite plaque de fermeture (20) et ladite couche de silicium (32), une cavité (38) destinée à être remplie de liquide,
ladite plaque de support (36) comportant au moins un conduit (102 ; 412, 412') la traversant de part en part,
ladite couche de matériau isolant (34) étant en oxyde de silicium et ayant au moins une zone entièrement exempte de matière (35) placée au moins dans le prolongement dudit conduit (102 ; 412, 412') et obtenue par structuration par attaque chimique par ledit' conduit (102 ;412,412'), afin de définir, avec ladite cavité (38) et dans ladite couche de silicium (32), un organe mobile (40) apte, grâce à son élasticité et sous la pression de liquide dans ladite cavité (38), à se rapprocher, de manière réversible, de ladite plaque de support (36).

3. Dispositif selon la revendication 2, **caractérisé en ce que** ledit organe mobile (40) se rapproche, de manière réversible, de ladite plaque de support (36)jusqu'à former un contact entre ledit organe mobile (40) et ladite plaque de support (36).

4. Dispositif selon la revendication 2 ou 3, **caractérisé en ce que** ladite plaque de fermeture (20) est en verre.

5. Dispositif selon l'une quelconque des revendications 2 à 4, **caractérisé en ce que** ladite plaque de support (36) est en silicium, en quartz ou en saphir.

6. Dispositif selon l'une quelconque des revendications 2 à 5, **caractérisé en ce que** ladite plaque de support (36) présente une épaisseur comprise entre 50 µm et 1 mm.

7. Dispositif selon la revendication 6, **caractérisé en ce que** ladite plaque de support (36) présente une épaisseur comprise entre 300 µm et 500 µm.

8. Dispositif selon l'une quelconque des revendications 2 à 7, **caractérisé en ce que** ladite couche de matériau isolant (34) présente une épaisseur comprise entre 100 nm et 2 µm.

9. Dispositif selon la revendication 8, **caractérisé en ce que** ladite couche de matériau isolant (34) présente une épaisseur comprise entre 0,5 µm et 1 µm.

10. Dispositif selon l'une quelconque des revendications 2 à 9, **caractérisé en ce que** ladite couche de silicium (32) présente une épaisseur comprise entre 1 µm et 100 µm.

11. Dispositif selon la revendication 10, **caractérisé en ce que** ladite couche de silicium (32) présente une épaisseur comprise entre 10 µm et 50 µm.

12. Dispositif fluidique selon l'une quelconque des revendications 2 à 11, **caractérisé en ce qu'**il constitue un organe de contrôle d'entrée de liquide (100; 100' ; 100" ; 100"') formant une valve anti-retour, ladite cavité (38) présentant au moins un dégagement (104; 104₁ ; 104₂) usiné dans toute l'épaisseur de la couche de silicium (32),
ledit conduit formé dans ladite plaque de support (36) constituant un conduit d'entrée de liquide (102) situé au moins en regard de ladite cavité (38),
ladite zone (35) entièrement exempte de matière s'étendant au moins dans le prolongement dudit dégagement (104 ; 104₁ ; 104₂), ledit organe mobile (40) formant un clapet pour ladite valve, une partie de ladite' couche de silicium (32) entourant ledit organe mobile (40) permettant, par son élasticité et par la différence de pression de liquide entre ledit conduit d'entrée de liquide (102) et ladite cavité (38), le mouvement réversible dudit organe mobile (40) en direction de ladite plaque de support (36).

13. Dispositif fluidique (100; 100') selon la revendication 12, **caractérisé en ce que** ledit conduit d'entrée de liquide (102) est situé à proximité mais pas au regard dudit dégagement (104), et **en ce que** ledit organe mobile (40) réalise un mouvement entre une position fermée, dans laquelle l'organe mobile (40) est en contact étanche contre ladite plaque de support (36) qui forme, au moins autour dudit conduit (102), un siège pour ladite valve, l'écoulement de liquide étant empêché entre ledit conduit d'entrée de liquide (102) et la cavité (38), et une position ouverte, de la valve dans laquelle l'organe mobile (40) n'étant plus en contact étanche contre la plaque de support (36) autour dudit conduit (102), l'organe mobile (40) permet l'écoulement du liquide depuis ledit conduit d'entrée de liquide (102) vers ledit dégagement (104).

14. Dispositif fluidique (100') selon la revendication 13, **caractérisé en ce qu'**il comporte en outre, entre ladite plaque de fermeture (20) et ledit organe mobile (40), des moyens d'appui (106, 110) plaçant ledit organe mobile (40), dans sa position de repos, dans ladite position fermée.

15. Dispositif fluidique (100") selon la revendication 12, **caractérisé en ce que** ladite plaque de fermeture (20) est une première plaque de fermeture (20), **en ce qu'**il comprend en outre une deuxième-plaque de fermeture (20') fixée sur la face de la plaque de support (36) opposée à ladite première plaque de fermeture (20) et munie d'un conduit (102"a) la traversant de part en part, **en ce qu'**une partie mobile (361) est réalisée dans la plaque de support (36), en regard et dans le prolongement de ladite cavité (38), dudit organe mobile (40) et dudit conduit (102"a), ladite partie mobile (361) étant située à proximité mais pas au regard dudit dégagement (104₂), un volume annulaire exempt de matière (102") étant usiné dans toute l'épaisseur de la plaque de support (36), en regard de ladite zone (35) entièrement exempte de matière de la couche de matériau isolant (34), en séparant ladite partie mobile (361) du reste de la plaque de support (36) et en formant ledit conduit d'entrée de liquide (102") qui communique avec ledit dégagement (104₂), **en ce que** ladite couche de matériau isolant (34) présente une zone de liaison (321) reliant de manière solidaire ladite partie mobile (361) audit organe mobile (40), et **en ce qu'**il comporte en outre un élément de valve (370) annulaire réalisé dans un matériau anti-adhésion, ledit élément de valve (370) étant soit situé sur la face de la deuxième plaque de fermeture (20') placée en regard de ladite partie mobile (361) de sorte que, lorsque ledit organe mobile (40) est le plus près possible de la plaque de support (36), la face de la partie mobile (361) tournée vers la deuxième plaque de fermeture (20') et la face de l'élément de valve (370) tournée vers la plaque de support (36) sont en contact étanche mettant ainsi l'organe de contrôle d'entrée de liquide (100") en position de fermeture ou soit sur la face de ladite partie mobile (361) placée en regard de la deuxième plaque de fermeture (20') de sorte que, lorsque ledit organe mobile (40) est le plus près possible de la plaque de support (36), la face de l'élément de valve (370) tournée vers la deuxième plaque de fermeture (20') et la face de la deuxième plaque de fermeture (20') tournée vers la plaque de support (36) sont en contact étanche mettant ainsi l'organe de contrôle d'entrée de liquide (100") en position de fermeture, dans ladite position de fermeture le passage de liquide depuis le conduit (102°a) de la deuxième plaque de fermeture (20') vers ledit conduit d'entrée de liquide (102") de la plaque de support (36) étant empêché.

16. Dispositif fluidique (100"') selon la revendication 12, **caractérisé en ce que** ladite plaque de fermeture (20) est une première plaque de fermeture (20), **en ce qu'**il comprend en outre une deuxième plaque de fermeture (20') fixée sur la face de la plaque de support (36) opposée à ladite première plaque de fermeture (20) et munie d'un conduit (102"a) la traversant de part en part, **en ce qu'**une partie mobile (361) annulaire est réalisée dans la plaque de support (36), en regard et dans le prolongement de ladite cavité (38) et dudit organe mobile (40), un premier volume annulaire (102"'a) exempt de matière étant usiné dans toute l'épaisseur de la plaque de support (36), en regard de ladite zone (35) entièrement exempte de matière de la couche de matériau isolant (34) et de ladite cavité (38), en séparant ladite partie mobile (361) du reste de la plaque de support (36), un deuxième volume cylindrique exempt de matière (102'") étant usiné dans toute l'épaisseur de la plaque de support (36) à l'emplacement de la partie mobile en formant ledit, conduit d'entrée de liquide (102) qui communique avec ledit dégagement (104), **en ce que** ladite couche de matériau isolant (34) présente une zone de liaison (321) reliant de manière solidaire ladite partie mobile (361) audit organe mobile (40) autour dudit conduit d'entrée de liquide (102) et dudit dégagement (104₁), et **en ce qu'**il comporte en outre un élément de valve (370) annulaire entourant ledit conduit d'entrée de liquide (102"'), réalisé dans un matériau anti-adhésion, ledit élément de valve (370) étant soit situé sur la face de la deuxième plaque de fermeture (20') placée en regard de ladite partie mobile (361) de sorte que, lorsque ledit organe mobile (40) est le plus près possible de la plaque de support (36), la face de la partie mobile (361) tournée vers la deuxième plaque de fermeture (20') et la face de l'élément de valve (370) tournée vers la plaque de support (36) sont en contact étanche mettant ainsi l'organe de contrôle d'entrée de liquide (100"') en position de fermeture, ou soit sur la face de ladite partie mobile (361) placée en regard de la deuxième plaque de fermeture (20') en verre de sorte que, lorsque ledit organe mobile (40) est le plus près possible de la plaque de support (36), la face de l'élément de valve (370) tournée vers la deuxième plaque de fermeture (20') et la face de la deuxième plaque de fermeture (20') tournée vers la plaque de support (36) sont en contact étanche mettant ainsi l'organe de contrôle d'entrée de liquide (100"') en position de fermeture, dans ladite position de fermeture le liquide arrivant dans ledit premier volume annulaire (102"'a) depuis le conduit (102"a) de la deuxième plaque de fermeture (20') est empêché de pénétrer dans ledit conduit d'entrée de liquide (102"') de la plaque de support (36).

17. Dispositif fluidique selon l'une quelconque des revendications 2 à 11, **caractérisé en ce qu'**il constitue un organe de détection de pression de liquide (400),
ledit conduit formé dans ladite plaque de support (36) étant situé en regard de ladite cavité (38),
ladite plaque de support en silicium (36) présentant une partie (414) en regard de l'organe mobile (40) formant un îlot séparé du reste de la plaque de support (36) par ledit conduit (412').

18. Dispositif fluidique (400) selon la revendication 17, **caractérisé en ce que** ledit organe mobile (40) est susceptible de passer d'une position ouverte à une position fermée dans laquelle l'organe mobile (40) est en contact physique avec ladite partie (414) située en regard de l'organe mobile (40) qui forme un îlot séparé du reste de la plaque de support (36) par ledit conduit (412') et qui forme une partie d'appui (414) de la plaque de silicium, ce contact physique pouvant être détecté électriquement.

19. Dispositif fluidique (400) selon la revendication 18, **caractérisé en ce qu'**il comporte en outre, entre la partie d'appui (414) et ladite couche de silicium (32), des moyens de solidarisation (416).

20. Dispositif fluidique (400"') selon la revendication 17, **caractérisé en ce que** ladite plaque de fermeture (20) est une première plaque de fermeture (20), **en ce qu'**il comprend en outre une deuxième plaque de fermeture (20') fixée sur la face de la plaque de support (36) opposée à ladite première plaque de fermeture (20), **en ce que** ladite partie qui forme un îlot séparé du reste de la plaque de support (36) constitue une partie mobile (461), et **en ce que** ladite couche de matériau isolant (34) présente une zone de liaison (321) reliant de manière solidaire ladite partie mobile (461) audit organe mobile (40).

21. Dispositif fluidique selon l'une quelconque des revendications 2 à 11, **caractérisé en ce qu'**il constitue une micropompe
ladite cavité (38) comportant une chambre de pompage (504),
ledit conduit formé dans ladite plaque de support (36) constituant un premier conduit (102, 508, 412, 412', 204) situé en regard de ladite cavité (38),
ladite zone entièrement exempte de matière constituant une première zone (35₁) entièrement exempte de matière, ledit organe mobile (40) constituant un premier organe mobile (40) apte, sous la pression de liquide dans ladite chambre de pompage (504), à se rapprocher, de manière réversible, de ladite plaque de support (36), ledit premier organe mobile (40) appartenant au clapet d'un organe de contrôle d'entrée de liquide (100),
ledit dispositif fluidique comprenant en outre une partie de pompage (502) comprenant des moyens de commande munis d'une membrane de pompage (506), pour provoquer une variation périodique du volume de la chambre de pompage (504), et des moyens de contrôle de sortie du liquide (100).

22. Dispositif fluidique selon la revendication 21, **caractérisé en ce que** ledit premier organe mobile (40) est apte à venir en contact étanche contre ladite plaque de support (36), ledit premier organe mobile (40) constituant le clapet dudit organe de contrôle d'entrée de liquide (100).

23. Dispositif fluidique selon la revendication 22, **caractérisé en ce que** ladite couche de matériau isolant (34) présente en outre une deuxième zone (35₄) entièrement exempte de matière qui définit, avec ladite cavité (38) et dans ladite couche de silicium (32), un deuxième organe mobile (40) apte, sous la pression de liquide dans ladite chambre de pompage, à se rapprocher de ladite plaque de support (36), ledit deuxième organe mobile (40) constituant le clapet d'un organe de contrôle de sortie de liquide (200),

24. Dispositif fluidique selon l'une quelconque des revendications 21 à 23, **caractérisé en ce que** ledit organe de contrôle d'entrée de liquide (100) est conforme à l'une quelconque des revendications 12 à 16.

25. Dispositif fluidique selon l'une quelconque des revendications 21 à 24, **caractérisé en ce qu'**il comporte en outre au moins un organe de détection de pression de liquide (400) selon l'une quelconque des revendications 17 à 20.

26. Dispositif fluidique selon l'une quelconque des revendications 21 à 25, **caractérisé en ce que** ladite plaque de fermeture (20) est une première plaque de fermeture (20) en verre.

27. Dispositif fluidique selon la revendication 26, **caractérisé en ce qu'**il comporte en outre une deuxième plaque de fermeture (20') en verre fixée sur la face de la plaque de support (36) opposée à ladite première plaque de fermeture (20) en verre.

28. Dispositif fluidique selon la revendication 27, **caractérisé en ce que** lesdits moyens de commande de la micropompe sont situés en regard de la chambre de pompage (504) et intégrés directement à la micropompe, par fixation sur la face de la deuxième plaque de fermeture (20') tournée en direction opposée à l'empilement (30).

29. Dispositif fluidique selon l'une quelconque des revendications 21 à 27, **caractérisé en ce que** lesdits moyens de commande de la micropompe sont externes à la micropompe et reliés indirectement à ladite membrane de pompage (506).

30. Dispositif fluidique selon l'une quelconque des revendications 21 à 29, **caractérisé en ce que** lesdits moyens de commande fonctionnent de manière piézo-électrique, électromagnétique ou pneumatique.

31. Dispositif fluidique selon l'une quelconque des revendications 21 à 28, **caractérisé en ce qu'**une partie mobile de pompage (514 ;514') est réalisée dans ladite plaque de support (36), en regard de la chambre de pompage (504), un volume annulaire exempt de matière (508) usiné dans ladite plaque de support (36) séparant la partie mobile de pompage (514 ;514') du reste de la plaque de support (36) et **en ce que** lesdits moyens de commande de la micropompe sont situés en regard de la chambre de pompage (504) et intégrés directement à la micropompe, par fixation à ladite partie mobile de pompage (514 ;514').

32. Dispositif fluidique selon la revendication 31, **caractérisé en ce que** la couche de silicium (32) forme ladite membrane de pompage (506) en regard de la chambre de pompage (504) et **en ce que** ladite partie mobile de pompage (514') est percée de part en part par un passage (540) dans lequel est susceptible d'être logée une tige de commande dont une extrémité est fixée à la membrane (506) et dont l'autre extrémité, forme une poignée.

33. Dispositif fluidique selon la revendication 31, **caractérisé en ce que** ladite partie mobile (514 ;514') intègre au moins un organe de détection de pression de liquide.

34. Dispositif fluidique selon la revendication 33, **caractérisé en ce que** ladite partie de pompage (502') est munie d'au moins deux détecteurs de pression de liquide (400') formant chacun un organe de détection de pression de liquide et qui sont régulièrement angulairement répartis au niveau de ladite partie mobile de pompage (514') qui est percée de part en part d'au moins deux séries de conduits (512').

35. Dispositif fluidique selon la revendication 33, **caractérisé en ce que** ladite partie de pompage (502") est munie d'un détecteur de pression de liquide annulaire (400") présentant des conduits (512') traversant de part en part ladite partie mobile (514') dans une zone annulaire.

36. Utilisation d'un dispositif fluidique selon l'une quelconque des revendications 21 à 35, comme pompe à usage médical de type « implantable ».

37. Utilisation d'un dispositif fluidique selon l'une quelconque des revendications 21 à 35, comme pompe à usage médical de type « externe ».

## Patentansprüche

1. Verfahren zur Herstellung einer fluidische Vorrichtung, **dadurch gekennzeichnet, daß** es die folgenden Schritte umfaßt:
- es wird eine Aufschichtung (30) bereitgestellt, die eine Tragplatte (36), eine einzige Schicht aus Isoliermaterial (34), welche die Tragplatte (36) wenigstens teilweise bedeckt, sowie eine Schicht (32) aus monokristallinem oder polykristallinem Silizium umfaßt, welche die genannte Schicht aus Isoliermaterial (34) bedeckt und eine freie Fläche aufweist,
- es wird wenigstens eine Verschlußplatte( (20) bereitgestellt,
- aus der genannten Verschlußplatte (20) und/oder aus der freien Fläche der Siliziumschicht (32) wird durch Photolithographie und chemisches Ätzen ein Hohlraum (38) gearbeitet,
- es wird wenigstens ein Kanal (102; 412, 412'), welcher die Tragplatte (36) vollständig durchquert, durch Photolithographie und chemisches Ätzen hergestellt,
- die genannte Schicht aus Isoliermaterial (34) wird wenigstens über den genannten Kanal (102; 412, 412') chemisch geätzt, so daß ein Bereich der genannten Siliziumschicht (32) von der Schicht aus Isoliermaterial (34) befreit ist, wodurch ein dem Hohlraum benachbartes bewegliches Element (40) gebildet wird, das geeignet ist, sich unter einem Flüssigkeitsdruck in dem genannten Hohlraum (38) der Tragplatte (36) reversibel zu nähern,
- die Verschlußplatte (20) wird durch ein physikalisch-chemisches Verfahren, vorzugsweise durch Plattenschweißen, auf dichte Weise mit der freien Fläche der Siliziumschicht (32) verbunden.

2. Fluidische Vorrichtung (100; 400; 500) umfassend eine Aufschichtung (30) vom Typ SILICON-ON-INSULATOR "SOI", die von einer Verschlußplatte (20) bedeckt ist,
wobei die Aufschichtung (30) eine Tragplatte (36), eine einzige Schicht aus Isoliermaterial (34), welche die Tragplatte (36) wenigstens teilweise bedeckt, sowie eine Schicht (32) aus monokristallinem oder polykristallinem Silizium umfaßt, welche die genannte Schicht aus Isoliermaterial (34) bedeckt und von der Verschlußplatte (20) bedeckt ist,
wobei die Verschlußplatte (20) und/oder die Siliziumschicht (32) bearbeitet wird bzw. werden, um zwischen der Verschlußplatte (20) und der Siliziumschicht (32) einen Hohlraum (38) zu bilden, der dazu bestimmt ist, mit Flüssigkeit gefüllt zu werden,
wobei die Tragplatte (36) wenigstens einen Kanal (102; 412, 412') aufweist, der sie vollständig durchquert,
wobei die Schicht aus lsoliermaterial (34) aus Siliziumoxid besteht und wenigstens einen vollkommen materialfreien Bereich (35) aufweist, der wenigstens in der Verlängerung des Kanals (102; 412, 412') gelegen ist und durch Strukturierung mittels chemischem Ätzen über den genannten Kanal (102; 412, 412') erhalten wird, um mit dem Hohlraum (38) und in der Siliziumschicht (32) ein bewegliches Element (40) zu bilden, das geeignet ist, sich aufgrund seiner Elastizität und unter dem Flüssigkeitsdruck in dem Hohlraum (38) der Tragplatte (36) reversibel zu nähern.

3. Vorrichtung nach Anspruch 2, **dadurch gekennzeichnet, daß** sich das bewegliche Element (40) der Tragplatte (36) reversibel nähert, bis ein Kontakt zwischen dem beweglichen Element (40) und der Tragplatte (36) hergestellt ist.

4. Vorrichtung nach Anspruch 2 oder 3, **dadurch gekennzeichnet, daß** die Verschlußplatte (20) aus Glas besteht.

5. Vorrichtung nach irgendeinem der Ansprüche 2 bis 4, **dadurch gekennzeichnet, daß** die Tragplatte (36) aus Silizium, aus Quarz oder aus Saphir besteht.

6. Vorrichtung nach irgendeinem der Ansprüche 2 bis 5, **dadurch gekennzeichnet, daß** die Tragplatte (36) eine Dicke aufweist, die zwischen 50 µm und 1 mm liegt.

7. Vorrichtung nach Anspruch 6, **dadurch gekennzeichnet, daß** die Tragplatte (36) eine Dicke zwischen 300 µm und 500 µm aufweist.

8. Vorrichtung nach irgendeinem der Ansprüche 2 bis 7, **dadurch gekennzeichnet, daß** die Schicht aus Isoliermaterial (34) eine Dicke zwischen 100 nm und 2 µm aufweist.

9. Vorrichtung nach Anspruch 8, **dadurch gekennzeichnet, daß** die Schicht aus Isoliermaterial (34) eine Dicke aufweist, die zwischen 0,5 µm und 1 µm liegt.

10. Vorrichtung nach irgendeinem der Ansprüche 2 bis 9, **dadurch gekennzeichnet, daß** die Siliziumschicht (32) eine Dicke zwischen 1 µm und 100 µm aufweist.

11. Vorrichtung nach Anspruch 10, **dadurch gekennzeichnet, daß** die Siliziumschicht (32) eine Dicke aufweist, die zwischen 10 µm und 50 µm liegt.

12. Fluidische Vorrichtung nach irgendeinem der Ansprüche 2 bis 11, **dadurch gekennzeichnet, daß** sie ein Element zur Kontrolle des Flüssigkeitseintritts (100; 100'; 100"; 100"') darstellt, das ein Rückschlagventil bildet, wobei der Hohlraum (38) wenigstens einen in die gesamte Dicke der Siliziumschicht (32) gearbeiteten Freiraum (104; 104₁; 104₂) aufweist,
wobei der in der Tragplatte (36) ausgebildete Kanal einen Flüssigkeitseintrittskanal (102) bildet, der wenigstens dem Hohlraum (38) gegenüberliegt,
wobei der vollständig materialfreie Bereich (35) sich wenigstens in der Verlängerung des Freiraums (104; 104₁; 104₂) erstreckt, wobei das bewegliche Element (40) eine Klappe für das genante Ventil bildet, wobei ein Teil der Siliziumschicht (32) das bewegliche Element (40) umgibt, das durch seine Elastizität und den Flüssigkeitsdruckunterschied zwischen dem Flüssigkeitseintrittskanal (102) und dem Hohlraum (38) die reversible Bewegung des beweglichen Elements (40) in Richtung auf die Tragplatte (36) ermöglicht.

13. Fluidische Vorrichtung (100; 100') nach Anspruch 12, **dadurch gekennzeichnet, daß** der Flüssigkeitseintrittskanal (102) in der Nähe des genannten Freiraums (104) gelegen ist, aber diesem nicht gegenüberliegt, und daß das bewegliche Element (40) zwischen einer geschlossenen Stellung, in welcher das bewegliche Element (40) dicht an der Tragplatte (36) anliegt, die wenigstens um den Kanal (102) herum einen Sitz für das Ventil bildet, wobei die Flüssigkeitsströmung zwischen dem Flüssigkeitseinirittskanal (102) und dem Hohlraum (38) verhindert wird, und einer geöffneten Stellung des Ventils, in der das bewegliche Element (40) nicht mehr in dichtem Kontakt mit der Tragplatte (36) um den Kanal (102) ist, wobei das bewegliche Element (40) das Strömen der Flüssigkeit von dem Flüssigkeitseintrittskanal (102) zu dem Freiraum (104) hin ermöglicht, eine Bewegung ausführt.

14. Fluidische Vorrichtung (100') nach Anspruch 13, **dadurch gekennzeichnet, daß** sie ferner zwischen der Verschlußplatte (20) und dem beweglichen Element (40) Stützmittel (106, 110) aufweist, die das bewegliche Element (40) in seiner Ruhestellung in die genannte geschlossene Position bringen.

15. Fluidische Vorrichtung (100") nach Anspruch 12, **dadurch gekennzeichnet, daß** die Verschlußplatte (20) eine erste Verschlußplatte (20) ist, daß sie außerdem eine zweite Verschlußplatte (20') aufweist, die an der der ersten Verschlußplatte (20) entgegengesetzten Seite der Tragplatte (36) befestigt ist und die mit einem sie vollständig durchquerenden Kanal (102"a) versehen ist, daß ein bewegliches Teil (361) in der Tragplatte (36) gegenüber und in der Verlängerung des Hohlraums (38), des beweglichen Elements (40) und des Kanals (102"a) ausgebildet ist, wobei das bewegliche Teil (361) in der Nähe des Freiraums (104₂) gelegen ist, aber diesem nicht gegenüberliegt, wobei ein materialfreier, ringförmiger Raum (102") in die gesamte Dicke der Tragplatte (36) gegenüber des vollständig materialfreien Bereichs (35) der Schicht aus Isoliermaterial (34) gearbeitet ist, wodurch das bewegliche Teil (361) von dem Rest der Tragplatte (36) getrennt und der Flüssigkeitseintrittskanal (102") gebildet wird, welcher mit dem Freiraum (104₂) in Verbindung steht, daß die Schicht aus lsoliermaterial (34) einen Verbindungsbereich (321) aufweist, welcher das bewegliche Teil (361) mit dem beweglichen Element (40) fest verbindet, und daß sie außerdem ein ringförmiges Ventilelement (370) aufweist, das aus einem adhäsionshemmenden Material gefertigt ist, wobei das Ventilelement (370) entweder auf der Seite der zweiten Verschlußplatte (20') gelegen ist, welche dem beweglichen Teil (361) gegenüberliegt, so daß dann, wenn sich das bewegliche Element (40) so nah wie möglich an der Tragplatte (36) befindet, die der zweiten Verschlußplatte (20') zugewandte Seite des beweglichen Teils (361) und die der Tragplatte (36) zugewandte Seite des Ventilelements (370) in dichtem Kontakt sind, wodurch das Element zur Kontrolle des Flüssigkeitseintritts (100") in die Schließposition bewegt wird, oder es auf der Seite des beweglichen Teils (361) gelegen ist, welche der zweiten Verschlußplatte (20') gegenüberliegt, so daß dann, wenn sich das bewegliche Element (40) so nah wie möglich an der Tragplatte (36) befindet, die der zweiten Verschlußplatte (20') zugewandte Seite des Ventilelements (370) und die der Tragplatte (36) zugewandte Seite der zweiten Verschlußplatte (20') in dichtem Kontakt sind, wodurch das Element zur Kontrolle des Flüssigkeitseintritts (100") in die Schließstellung bewegt wird, wobei in der genannten Schließstellung der Flüssigkeitsdurchlaß von dem Kanal (102"a) der zweiten Verschlußplatte (20') zu dem Flüssigkeitseintrittskanal (102") der Tragplatte (36) verhindert wird.

16. Fluidische Vorrichtung (100"') nach Anspruch 12, **dadurch gekennzeichnet, daß** die Verschlußplatte (20) eine erste Verschlußplatte (20) ist, daß sie ferner eine zweite Verschlußplatte (20') aufweist, die an der der ersten Verschlußplatte (20) entgegengesetzten Seite der Tragplatte (36) befestigt ist und die mit einem sie vollständig durchquerenden Kanal (102"a) versehen ist, daß ein ringförmiges bewegliches Teil (361) in der Tragplatte (36) gegenüber und in der Verlängerung des Hohlraums (38) und des beweglichen Elements (40) ausgebildet ist, wobei ein erster materialfreier, ringförmiger Raum (102"'a) in die gesamte Dicke der Tragplatte (36) gegenüber des vollständig materialfreien Bereichs (35) der Schicht aus Isoliermaterial (34) und des Hohlraums (38) gearbeitet ist, wodurch das bewegliche Teil (361) von dem Rest der Tragplatte (36) getrennt wird, wobei ein zweiter zylindrischer, materialfreier Raum (102"') in die gesamte Dicke der Tragplatte (36) an der Stelle des beweglichen Teils gearbeitet ist, wodurch der Flüssigkeitseintrittskanal (102) gebildet wird, welcher mit dem Freiraum (104) in Verbindung steht, daß die Schicht aus Isoliermaterial (34) einen Verbindungsbereich (321) aufweist, welcher das bewegliche Teil (361) mit dem beweglichen Element (40) um den Flüssigkeitseintrittskanal (102) und den Freiraum (104₁) fest verbindet, und daß sie außerdem ein ringförmiges Ventilelement (370) aufweist, das den Flüssigkeitseintrittskanal (102"') umgibt und aus einem adhäsionshemmenden Material gefertigt ist, wobei das Ventilelement (370) entweder auf der Seite der zweiten Verschlußplatte (20') gelegen ist, welche dem beweglichen Teil (361) gegenüberliegt, so daß dann, wenn sich das bewegliche Element (40) so nah wie möglich an der Tragplatte (36) befindet, die der zweiten Verschlußplatte (20') zugewandte Seite des beweglichen Teils (361) und die der Tragplatte (36) zugewandte Seite des Ventilelements (370) in dichtem Kontakt sind, wodurch das Element zur Kontrolle des Flüssigkeitseintritts (100"') in die Schließposition bewegt wird, oder es auf der Seite des beweglichen Teils (361) gelegen ist, welche der zweiten Verschlußplatte aus Glas (20') gegenüberliegt, so daß dann, wenn sich das bewegliche Element (40) so nah wie möglich an der Tragplatte (36) befindet, die der zweiten Verschlußplatte (20') zugewandte Seite des Ventilelements (370) und die der Tragplatte (36) zugewandte Seite der zweiten Verschlußplatte (20') in dichtem Kontakt sind, wodurch das Element zur Kontrolle des Flüssigkeitseintritts (100"') in die Schließstellung bewegt wird, wobei in der genannten Schließstellung die Flüssigkeit, welche von dem Kanal (102"a) der zweiten Verschlußplatte (20') in den ersten ringförmigen Raum (102"'a) gelangt, daran gehindert wird, in den Flüssigkeitseintrittskanal (102"') der Tragplatte (36) einzudringen.

17. Fluidische Vorrichtung nach irgendeinem der Ansprüche 2 bis 11, **dadurch gekennzeichnet, daß** sie ein Element zum Erfassen des Flüssigkeitsdrucks (400) bildet,
wobei der in der Tragplatte (36) ausgebildete Kanal dem Hohlraum (38) gegenüberliegt,
wobei die Siliziumtragplatte (36) einen dem beweglichen Element (40) gegenüberliegenden Teil (414) aufweist, der eine durch den Kanal (412') von der übrigen Tragplatte (36) getrennte Insel bildet.

18. Fluidische Vorrichtung (400) nach Anspruch 17, **dadurch gekennzeichnet, daß** das bewegliche Element (40) geeignet ist, von einer geöffneten Stellung in eine geschlossene Stellung überzugehen, in der das bewegliche Element (40) mit dem dem beweglichen Element (40) gegenüberliegenden Teil (414), das eine durch den Kanal (412') von der übrigen Tragplatte (36) getrennte Insel und einen Stützteil (414) der Siliziumplatte bildet, in physikalischem Kontakt ist, wobei dieser physikalische Kontakt elektrisch erfaßt werden kann.

19. Fluidische Vorrichtung (400) nach Anspruch 18, **dadurch gekennzeichnet, daß** sie außerdem zwischen dem Stützteil (414) und der Siliziumschicht (32) Mittel zum festen Verbinden (416) aufweist.

20. Fluidische Vorrichtung (400"') nach Anspruch 17, **dadurch gekennzeichnet, daß** die Verschlußplatte (20) eine erste Verschlußplatte (20) ist, daß sie ferner eine zweite Verschlußplatte (20') aufweist, die an der der ersten Verschlußplatte (20) entgegengesetzten Seite der Tragplatte (36) befestigt ist, daß der Teil, welcher eine Insel bildet, die von der übrigen Tragplatte (36) getrennt ist, einen beweglichen Teil (461) bildet, und daß die genannte Schicht aus Isoliermaterial (34) einen Verbindungsbereich (321) aufweist, welcher den beweglichen Teil (461) mit dem beweglichen Element (40) fest verbindet.

21. Fluidische Vorrichtung nach irgendeinem der Ansprüche 2 bis 11, **dadurch gekennzeichnet, daß** sie eine Mikropumpe bildet,
wobei der Hohlraum (38) eine Pumpkammer (504) aufweist,
wobei der in der Tragplatte (36) ausgebildete Kanal einen ersten Kanal (102, 508, 412, 412', 204) bildet, welcher dem genannten Hohlraum (38) gegenüberliegt,
wobei der vollständig materialfreie Bereich einen ersten vollständig materialfreien Bereich (35₁) bildet, wobei das bewegliche Element (40) ein erstes bewegliches Element (40) bildet, das geeignet ist, sich unter dem Flüssigkeitsdruck in der Pumpkammer (504) der Tragplatte (36) reversibel zu nähern, wobei das erste bewegliche Element (40) zu der Klappe eines Elements zur Kontrolle des Flüssigkeitseintritts (100) gehört,
wobei die Fluidische Vorrichtung ferner einen Pumpteil (502) aufweist, der Steuermittel, die mit einer Pumpmembran (506) versehen sind, um eine periodische Änderung des Volumens der Pumpkammer (504) zu bewirken, sowie Mittel zur Kontrolle des Flüssigkeitsaustritts (100) umfaßt.

22. Fluidische Vorrichtung nach Anspruch 21, **dadurch gekennzeichnet, daß** das erste bewegliche Element (40) geeignet ist, an der Tragplatte (36) in dichten Kontakt zu gelangen, wobei das erste bewegliche Element (40) die Klappe des genannten Elements zur Kontrolle des Flüssigkeitseintritts (100) bildet.

23. Fluidische Vorrichtung nach Anspruch 22, **dadurch gekennzeichnet, daß** die Schicht aus Isoliermaterial (34) außerdem einen zweiten, vollständig materialfreien Bereich (35₄) aufweist, der mit dem Hohlraum (38) und in der Siliziumschicht (32) ein zweites bewegliches Element (40) definiert, das geeignet ist, sich unter dem Flüssigkeitsdruck in der Pumpkammer der Tragplatte (36) zu nähern, wobei das zweite bewegliche Element (40) die Klappe eines Elements zur Kontrolle des Flüssigkeitsaustritts (200) bildet.

24. Fluidische Vorrichtung nach irgendeinem der Ansprüche 21 bis 23, **dadurch gekennzeichnet, daß** das Element zur Kontrolle des Flüssigkeitseintritts (100) mit irgendeinem der Ansprüche 12 bis 16 übereinstimmt.

25. Fluidische Vorrichtung nach irgendeinem der Ansprüche 21 bis 24, **dadurch gekennzeichnet, daß** sie ferner wenigstens ein Element zur Erfassung des Flüssigkeitsdrucks (400) nach irgendeinem der Ansprüche 17 bis 20 aufweist.

26. Fluidische Vorrichtung nach irgendeinem der Ansprüche 21 bis 25, **dadurch gekennzeichnet, daß** die Verschlußplatte (20) eine erste Verschlußplatte (20) aus Glas ist.

27. Fluidische Vorrichtung nach Anspruch 26, **dadurch gekennzeichnet, daß** sie darüber hinaus eine zweite Verschlußplatte (20') aus Glas aufweist, die an der der ersten aus Glas bestehenden Verschlußplatte (20) entgegengesetzten Seite der Tragplatte (36) befestigt ist.

28. Fluidische Vorrichtung nach Anspruch 27, **dadurch gekennzeichnet, daß** die Steuermittel der Mikropumpe der Pumpkammer (504) gegenüberliegen und durch Befestigung an der in entgegengesetzte Richtung zu der Aufschichtung (30) gewandten Seite der zweiten Verschlußplatte (20') direkt in die Mikropumpe integriert sind.

29. Fluidische Vorrichtung nach irgendeinem der Ansprüche 21 bis 27, **dadurch gekennzeichnet, daß** die Steuermittel der Mikropumpe außerhalb der Mikropumpe angeordnet und indirekt mit der Pumpmembran (506) verbunden sind.

30. Fluidische Vorrichtung nach irgendeinem der Ansprüche 21 bis 29, **dadurch gekennzeichnet, daß** die Steuermittel piezo-elektrisch, elektromagnetisch oder pneumatisch arbeiten.

31. Fluidische Vorrichtung nach irgendeinem der Ansprüche 21 bis 28, **dadurch gekennzeichnet, daß** ein bewegliches Pumpteil (514; 514') in der Tragplatte (36) gegenüber der Pumpkammer (504) ausgebildet ist, wobei ein in die Tragplatte (36) gearbeiteter, materialfreier, ringförmiger Raum (508) das bewegliche Pumpteil (514; 514') von der übrigen Tragplatte (36) trennt, und daß die Steuermittel der Mikropumpe der Pumpkammer (504) gegenüberliegen und durch Befestigung an dem beweglichen Pumpteil (514; 514') direkt in die Mikropumpe integriert sind.

32. Fluidische Vorrichtung nach Anspruch 31, **dadurch gekennzeichnet, daß** die Siliziumschicht (32) die Pumpmembran (506) gegenüber der Pumpkammer (504) bildet und daß das bewegliche Pumpteil (514') vollkommen von einem Durchgang (540) durchzogen ist, in dem ein Steuerstift gelagert werden kann, dessen eines Ende an der Membran (506) befestigt ist und dessen anderes Ende einen Griff bildet.

33. Fluidische Vorrichtung nach Anspruch 31, **dadurch gekennzeichnet, daß** das bewegliche Teil (514; 514') wenigstens ein Element zur Erfassung des Flüssigkeitsdrucks beinhaltet.

34. Fluidische Vorrichtung nach Anspruch 33, **dadurch gekennzeichnet, daß** das Pumpteil (502') mit wenigstens zwei Flüssigkeitsdruckfühlern (400') ausgestattet ist, die jeweils ein Element zur Erfassung des Flüssigkeitsdrucks bilden und die im Bereich des beweglichen Pumpteils (514'), das von wenigstens zwei Reihen von Kanälen (512') vollkommen durchzogen ist, gleichmäßig winkelmäßig verteilt sind.

35. Fluidische Vorrichtung nach Anspruch 33, **dadurch gekennzeichnet, daß** das Pumpteil (502") mit einem ringförmigen Flüssigkeitsdruckfühler (400") ausgestattet ist, der Kanäle (512') aufweist, welche das bewegliche Teil (514') in einem ringförmigen Bereich vollständig durchqueren.

36. Verwendung einer Fluidische Vorrichtung nach irgendeinem der Ansprüche 21 bis 35 als Pumpe für den medizinischen Gebrauch vom Typ "implantierbar".

37. Verwendung einer Fluidische Vorrichtung nach irgendeinem der Ansprüche 21 bis 35 als Pumpe für den medizinischen Gebrauch vom Typ "extern".

## Claims

1. A method of manufacturing a fluid-flow device, the method being **characterised in that** it comprises the following steps:
- providing a stack (30) comprising a support wafer (36), a single layer of insulating material (34) covering at least part of said support wafer (36), and a layer of single-crystal or polycrystalline silicon (32) covering said layer of insulating material (34) and presenting a free face;
- providing at least one closure wafer (20);
- using photolithography and chemical etching to machine a cavity (38) from said closure wafer (20) and/or from the free face of said silicon layer (32);
- using photolithography and chemical etching to machine at least one duct (102; 412, 412') passing right through said support wafer (36);
- chemically etching said layer of insulating material (34) at least via said duct (102; 412, 412') such that a zone of said silicon layer (32) is freed from said layer of insulating material (34), thereby forming a moving member (40) that is adjacent to said cavity and that responds to liquid pressure in said cavity (38) by moving reversibly towards said support wafer (36); and
- using a physicochemical method, preferably by wafer bonding, to connect said closure wafer (20) in leaktight manner to said free face of silicon layer (32).

2. A fluid-flow device (100; 400; 500) comprising a stack (30) of the silicon-on-insulator (SOI) type covered by a closure wafer (20), said stack (30) comprising a support wafer (36), a single layer of insulating material (34) covering at least part of said support wafer (36), and a layer of single-crystal or polycrystalline silicon (32) covering said layer of insulating material (34) and covered by said closure wafer (20),
said closure wafer (20) and/or said silicon layer (32) being machined so as to define between said closure wafer (20) and said silicon layer (32), a cavity (38) to be filled with liquid,
said support wafer (36) having at least one duct (102; 412, 412') passing right through it, and
said layer of insulating material (34) being made of silicon oxide and having at least one zone that is entirely free from material (35) placed at least in line with said duct (102; 412, 412') and obtained by structuring by chemical etching via said duct (102; 412, 412') so as to co-operate with said cavity (38) to define a moving member (40) in said silicon layer (32) that responds, by virtue of its elasticity, to pressure of the liquid in said cavity (38) by moving reversibly towards said support wafer (36).

3. A device according to claim 2, **characterised in that** said moving member (40) moves reversibly towards said support wafer (36) until making contact between said moving member (40) and said support wafer (36).

4. A device according to claim 2 or claim 3, **characterised in that** said closure wafer (20) is made of glass.

5. A device according to any one of claims 2 to 4, **characterised in that** said support wafer (36) is made of silicon, of quartz, or of sapphire.

6. A device according to any one of claims 2 to 5, **characterised in that** said support wafer (36) is of thickness lying in the range 50 µm to 1 mm.

7. A device according to claim 6, **characterised in that** said support wafer (36) is of thickness lying in the range 300 µm to 500 µm.

8. A device according to any one of claims 2 to 7, **characterised in that** said layer of insulating material (34) is of thickness lying in the range 100 nm to 2 µm.

9. A device according to claim 8, **characterised in that** said layer of insulating material (34) presents thickness lying in the range 0.5 µm to 1 µm.

10. A device according to any one of claims 2 to 9, **characterised in that** said silicon layer (32) presents a thickness lying in the range 1 µm to 100 µm.

11. A device according to claim 10, **characterised in that** said silicon layer (32) presents a thickness lying in the range 10 µm to 50 µm.

12. A fluid-flow device according to any one of claims 2 to 11, **characterised in that** it constitutes a liquid inlet control member (100; 100'; 100"; 1001"') forming a non-return check valve, said cavity (38) presenting at least one gap (104; 104₁; 104₂) machined in the entire thickness of the silicon layer (32),
said duct formed in said support wafer (36) constituting a liquid inlet duct (102) situated at least in register with said cavity (38), and
said zone (35) entirely free of material extending at least in line with said gap (104; 104₁; 104₂), said moving member (40) forming a flap for said valve, a portion of said silicon layer (32) surrounding said moving member (40) presenting elasticity making it possible in the event of a difference in liquid pressure between said liquid inlet duct (102) and said cavity (38) to allow said moving member (40) to move reversibly towards said support wafer (36).

13. A fluid-flow device (100; 100') according to claim 12, **characterised in that** said liquid inlet duct (102) is situated close to but not in register with said gap (104), and **in that** said moving member (40) moves between a closed position in which the moving member (40) is in leaktight contact against said support wafer (36) which forms a seat for said valve at least: around said duct (102), liquid flow being prevented between said liquid inlet duct (102) and the cavity (38), and an open position of the valve in which the moving member (40) is no longer in leaktight contact against the support wafer (36) around said duct (102), in which the moving member (40) allows liquid flow from said liquid inlet duct (102) towards said gap (104).

14. A fluid-flow device (100') according to claim 13, **characterised in that** it further comprises bearing means (106, 110) between said closure wafer (20) and said moving member (40), said bearing means (106, 110) placing said moving member (40) in said closed position while it is in its rest position.

15. A fluid-flow device (100") according to claim 12, **characterised in that** said closure wafer (20) is a first closure wafer (20), **in that** it further comprises a second closure wafer (20') fixed on the face of the support wafer (36) facing away from said first closure wafer (20) and provided with a duct (102"a) passing right through it, **in that** a moving portion (361) is made in the support wafer (36) in register with and extending said cavity (38), said moving member (40), and said duct (102"a), said moving portion (361) being situated close to but not in register with said gap (104₂), an annular volume free from material (102") being machined through the entire thickness of the support wafer (36) in register with said zone (35) that is entirely free from material in the layer of insulating material (34), thereby separating said moving portion (361) from the remainder of the support wafer (36) and forming said liquid inlet duct (102") which communicates with said gap (104₂), **in that** said layer of insulating material (34) presents a connection zone (321) securely connecting said moving portion (361) to said moving member (40), and **in that** it further comprises an annular valve element (370) made in an anti-adhesion material, said valve element (370) being situated either on the face of the second closure wafer (20') placed facing said moving portion (361) so that when said moving member (40) is as close as possible to the support wafer (36), the face of the moving portion (361) facing towards the second closure wafer (20') and the face of the valve element (370) facing towards the support wafer (36) are in leaktight contact thus putting the liquid inlet control member (100") in its closed position, or else on the face of said moving portion (361) placed facing the second closure wafer (20) such that when said moving member (40) is as close as possible to the support wafer (36), the face of the valve element (370) facing towards said second closure wafer (20') and the face of the second closure wafer (20') facing towards the support wafer (36) are in leaktight contact, thus putting the liquid inlet control member (100") into the closed position, in which closed position flow of liquid from the duct (102"a) of the second closure wafer (20') towards said liquid inlet duct (102") of the support wafer (36) is prevented.

16. A fluid-flow device (100") according to claim 12, **characterised in that** said closure wafer (20) is a first closure wafer (20), **in that** it further comprises a second closure wafer (20') fixed on the face of the support wafer (36) facing away from said first closure wafer (20) and provided with a duct (102"a) passing right through it, **in that** the annular moving portion (361) is made in the support wafer (36) in register and in line with said cavity (38) and said moving member (40), a first annular volume (102"'a) free form material being machined through the entire thickness of the support wafer (36) in register with said cavity (38) and said zone (35) entirely free of material in the layer of insulating material (34), thereby separating said moving portion (361) from the remainder of the support wafer (36), a second cylindrical volume free from material (102"') being machined throughout the entire thickness of the support wafer (36) at the location of the moving portion, thereby forming said liquid inlet duct (102) which communicates with said gap (104), **in that** said layer of insulating material (34) presents a connection zone (321) securely connecting said moving portion (361) to said moving member (40) around said liquid inlet duct (102) and said gap (104₁), and **in that** it further comprises an annular valve element (370) surrounding said liquid inlet duct (102"'), and made of an anti-adhesion material, said valve element (370) being situated either on the face of the second closure wafer (20') placed facing said moving portion (361) so that when said moving member (40) is as close as possible to the support wafer (36), the face of the moving portion (361) facing towards the second closure wafer (20') and the face of the valve element (370) facing towards the support wafer (36) are in leaktight contact thus putting the liquid inlet control member (100"') into its closed position, or else on the face of said moving portion (361) placed facing the second glass closure wafer (20') such that when said moving member (40) is as close as possible to the support wafer (36), the face of the valve element (370) facing towards the second closure wafer (20') and the face of the second closure wafer (20') facing towards the support wafer (36) are in leaktight contact thus putting the liquid inlet control member (100"') in its closed position, in which closed position liquid arriving in said first annular volume (102"'a) from the duct (102"a) in the second closure wafer (20') is prevented from penetrating into said liquid inlet duct (102"') in the support wafer (36).

17. A fluid-flow device according to any one of claims 2 to 11, **characterised in that** it constitutes a liquid pressure detection member (400),
said duct formed in said support wafer (36) being situated in register with said cavity (38), and
said silicon support wafer (36) presenting a portion (414) in register with the moving member (40) forming an island that is separated from the remainder of the support wafer (36) by said duct (412').

18. A fluid-flow device (400) according to claim 17, **characterised in that** said moving member (40) is capable of going from an open position to a closed position in which the moving member (40) is in physical contact with said portion (414) situated facing the moving member (40) forming an island that is separated from the remainder of the support wafer (36) by said duct (412') and which forms a bearing portion (414) of the silicon wafer, said physical contact being electrically detectable.

19. A fluid-flow device (400) according to claim 18, **characterised in that** it further comprises connection means (416) between the bearing portion (414) and said silicon layer (32).

20. A fluid-flow device (400"') according to claim 17, **characterised in that** said closure wafer (20) is a first closure wafer (20), **in that** it further comprises a second closure wafer (20') fixed on the face of the support wafer (36) facing away from said first closure wafer (20), **in that** said portion which forms an island separated from the remainder of the support wafer (36) constitutes a moving portion (461), and **in that** said layer of insulating material (34) presents a connection zone (321) connecting said moving portion (461) securely to said moving member (40).

21. A filuid-flow device according to any one of claims 2 to 11, **characterised in that** it constitutes a micropump,
said cavity (38) including a pump chamber (504),
said duct formed in said support wafer (36) constituting a first duct (102, 508, 412, 412', 204) situated in register with said cavity (38),
said zone that is entirely free of material constituting a first zone (35₁) that is entirely free of material, said moving member (40) constituting a first moving member (40) that is suitable under pressure of liquid in said pump chamber (504) for moving reversibly towards said support wafer (36), said first moving member (40) forming part of the flap of a liquid inlet control member (100), and
said fluid-flow device further comprising a pumping portion (502) comprising control means fitted with a pump diaphragm (506) to cause the volume of the pump chamber (504) to vary periodically, and liquid outlet control means (100).

22. A fluid-flow device according to claim 21, **characterised in that** said first moving member (40) is suitable for coming into leaktight contact against said support wafer (36), said first moving member (40) constituting the flap of said liquid inlet control member (100).

23. A fluid-flow device according to claim 22, **characterised in that** said layer of insulating material (34) further presents a second zone (35₄) that is entirely free of material which co-operates with said cavity (38) to define a second moving member (40) in said silicon layer (32), the second moving member (40) being suitable under the pressure of liquid in said pump chamber for moving towards said support wafer (36), said second moving member (40) constituting the flap of a liquid outlet control member (200).

24. A fluid-flow device according to any one of claims 21 to 23, **characterised in that** said liquid inlet control member (100) is in accordance with any one of claims 12 to 16.

25. A fluid-flow device according to any one of claims 21 to 24, **characterised in that** it further comprises at least one liquid pressure detection member (400) in accordance with any one of claims 17 to 20.

26. A fluid-flow device according to any one of claims 21 to 25, **characterised in that** said closure wafer (20) is a first closure wafer (20) made of glass.

27. A fluid-flow device according to claim 26, **characterised in that** it further comprises a second closure wafer (20') made of glass fixed on the face of the support wafer (36) facing away from said first closure wafer (20) made of glass.

28. A fluid-flow device according to claim 27, **characterised in that** said micropump control means are situated facing the pump chamber (504) and are integrated directly in the micropump by being fixed to the face of the second closure wafer (20') facing away from the stack (30).

29. A fluid-flow device according to any one of claims 21 to 27, **characterised in that** said micropump control means are external to the micropump and are connected indirectly to said pump diaphragm (506).

30. A fluid-flow device according to any one of claims 21 to 29, **characterised in that** said control means operate in piezoelectric, electromagnetic, or pneumatic manner.

31. A fluid-flow device according to any one of claims 21 to 28, **characterised in that** a moving pumping portion (514; 514') is made in said support wafer (36) in register with the pump chamber (504), an annular volume free of material (508) machined in said support wafer (36) separating the moving pump portion (514; 514') from the remainder of the support wafer (36), and **in that** said micropump control means are situated in register with the pump chamber (504) and directly integrated in the micropump by being fixed to said moving pump portion (514; 514').

32. A fluid-flow device according to claim 31, **characterised in that** the silicon layer (32) forms said pump diaphragm (506) in register with the pump chamber (504), and **in that** said moving pump portion (514') is pierced right through by a passage (540) which is suitable for receiving a control rod having one end fixed to the diaphragm (506) and having its opposite end forming a handle.

33. A fluid-flow device according to claim 31, **characterised in that** said moving portion (514; 514') includes at least one liquid pressure detection member.

34. A fluid-flow device according to claim 33, **characterised in that** said pumping portion (502') is provided with at least two liquid pressure detectors (400') each forming a liquid pressure detection member, said members being regularly distributed angularly in said moving pumping portion (514') which is pieced right through by at least two series of ducts (512').

35. A fluid-flow device according to claim 33, **characterised in that** said pumping portion (502") is provided with an annular liquid pressure detector (400") presenting ducts (512') passing right through said moving portion (514') in an annular zone.

36. The use of a fluid-flow device according to any one of claims 21 to 35, as a medical pump of the "implantable" type.

37. The use of a fluid-flow device according to any one of claims 21 to 35, as a medical pump of the "external" type.
